(19) Europäisches Patentamt
European Patent Office
Office européen des brevets

(11) **EP 1 768 677 B1**

(12) **EUROPEAN PATENT SPECIFICATION**

(45) Date of publication and mention
of the grant of the patent:
**25.06.2008 Bulletin 2008/26**

(51) Int Cl.:
***A61K 31/713*** (2006.01)

(21) Application number: **05773148.1**

(86) International application number:
**PCT/EP2005/007198**

(22) Date of filing: **04.07.2005**

(87) International publication number:
**WO 2006/002971 (12.01.2006 Gazette 2006/02)**

(54) **NUCLEIC ACIDS FOR THE TREATMENT OF HMGB1-RELATED PATHOLOGIES**

NUKLEINSÄUREN ZUR BEHANDLUNG VON ERKRANKUNGEN IM ZUSAMMENHANG MIT HMGB1

AGENTS THERAPEUTIQUES POUR TRAITER DES PATHOLOGIES ASSOCIEES A LA HMGB1

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.07.2004 US 584678 P**
**26.01.2005 US 646586 P**

(43) Date of publication of application:
**04.04.2007 Bulletin 2007/14**

(73) Proprietor: **Creabilis Therapeutics S.P.A.**
**10010 Colleretto Giacosa (IT)**

(72) Inventors:
• **BARONE, Domenico, G.**
**I-10129 Torino (IT)**
• **BIANCHI, Marco, E.**
**I-20068 Peschiera Borromeo (IT)**
• **BUCCI, Enrico, M.**
**I-80134 Napoli (IT)**
• **FUMERO, Silvano**
**I-10015 Ivrea (IT)**
• **VALENTE, Margherita**
**I-04023 Formia (IT)**
• **SAPIO, Roberto**
**I-84092 Bellizzi (IT)**
• **MUSUMECI, Domenica**
**I-81030 Sant'Arpino (IT)**

(74) Representative: **Weiss, Wolfgang et al**
**Weickmann & Weickmann**
**Patentanwälte**
**Postfach 86 08 20**
**81635 München (DE)**

(56) References cited:
**WO-A-94/09158        WO-A-02/074337**
**WO-A-20/04046345**

• **MÜLLER S; BIANCHI M; KNAPP S.: "Thermodynamics of HMGB1 interaction with duplex DNA" BIOCHEMISTRY, vol. 40, no. 34, 2001, pages 10254-10261, XP002359280**

**Description**

The present invention relates to the use of synthetic double-stranded nucleic acid or nucleic acid analogue molecules with a bent shape structure for the prevention and treatment of pathologies induced directly or indirectly by the HMGB1 protein.

Recent researches in the field of sepsis and inflammation have led to an improved understanding of the pathogenic mechanisms and events underlying their clinical onset and development. In the early stages of sepsis, for instance, bacterial endotoxins stimulate cells of the innate immune system which release pro-inflammatory cytokines (TNF, IL-1$\alpha$ and IL-6) These early cytokines, in turn, induce the release of a later-acting downstream mediator - identified as the known protein HMGB1 - that triggers the pathological sequelae mediated by the subsequent release of cytokines like TNF, IL-1$\alpha$, IL-1$\beta$, IL-1Ra, IL-6, IL-8, etc., leading to a multisystem pathogenesis or to a lethal systemic inflammation (Andersson et al., 2002).

The HMGB1 protein belongs to the family of high mobility group (HMG) proteins. HMG proteins, so called due to their high electrophoretic mobility in polyacrylamide gels, are the most ubiquitous non-histone proteins associated with isolated chromatin in eukaryotic cells. These proteins play a generalized "architectural" role in DNA bending, looping, folding and wrapping since they either distort, bend or modify DNA structures complex with transcription factors or histones (Andersson et al., 2000; Agresti et al., 2003; Degryse et al., 2003). The high mobility group 1 (HMGB1) protein is usually a nuclear factor, in particular a transcriptional regulatory molecule causing DNA bending and facilitating the binding of several transcriptional complexes.

Structurally, the HMGB1 protein is a ca. 25 kDa protein with a highly conserved sequence among mammals, whereby 2 out of 214 amino acids have conservative substitutions in all mammalian species. HMGB1 is ubiquitously present in all vertebrate nuclei and, in particular, can be found in fibroblasts, neurons, hepatocytes, glia and in cells derived from hematopoietic stem cells, including monocytes/macrophages, neutrophils and platelets.. The HMGB1 molecule has a tripartite structure composed of three distinct domains: two DNA binding domains called HMG Box A and Box B, and an acid carboxyl terminus, making it bipolarly charged.

The two HMGB boxes are involved in the protein's function as a non-sequence-specific architectural DNA-binding element, conferring the ability to bind DNA into recognised distorted DNA structures and which stabilize nucleosome assembly, remodelling and sliding. Both the A- and B-HMG boxes are made up of highly conserved 80 amino acid residues, are strongly positively charged and are arranged in three $\alpha$-helix having a similar L-shaped fold. The long arm of the "L" contains the N-terminal extended strand and helix III (Andersson et al. 2002; Agresti et al., 2003; Thomas, J. O. 2001), while the short arm comprises helices I and II. Structure-function analysis reveals that the B-box of HMGB1 contains the proinflammatory cytokine domain.

The third domain, the carboxyl terminus or acidic tail, is extremely negatively charged since it contains 30 repetitive aspartic and glutamic acid residues, and is linked to the boxes by a basic region of about 20 residues. Mouse and rat HMGB1 differ from the human form by only two substitutions that are located in this continuous C-terminal stretch.

HMGB1 binds rather weakly to the B-form variety of linear double-stranded DNA with no sequence specificity, while it binds in the interior of the nucleus with high affinity to supercoiled DNA, to unusual DNA structures like 4-way junctions (cruciform DNA), bulged DNA and bent DNA (Ferrari et al., 1992; Pontiggia et al., 1993).

Besides its nuclear location and role as a transcription factor regulator, HMGB1 has also been found in the extracellular medium, actively released by activated cells of the immune systems (monocytes and macrophages) or passively released by damaged or necrotic cells (Andersson et al., 2002; Scaffidi et al., 2002; Bonaldi et a., 2002; Taniguchi et al., 2003; Palumbo et al., 2004; Friedman et al., 2003).

Extracellularly released HMGB1 acts as a potent cytokine and as an extremely potent macrophage-stimulating factor. HMGB1 acts directly by binding to the cell membrane inducing signaling and chemotaxis, having a chemokine-like function (Yang et al., 2001), and further acting indirectly by up-regulating the expression and secretion of pro-inflammatory cytokines. This makes extracellular HMGB1 protein a potent chemotactic and immunoregulatory protein which promotes an effective inflammatory immune response.

Furthermore, other proteins belonging to the family of HMG-proteins and able to bend DNA are released together with HMGB1 in the extracellular medium. These proteins are inter alia HMGB2, HMGB3, HMG-1L10, HMG-4L and SP100-HMG. They share with HMGB1 highly homologous amino acid sequences. Like HMGB1, they trigger/sustain inflammatory pathologies interacting with the same receptors and leading to the same downstream pathways of interaction.

In healthy cells, HMGB1 migrates to the cytoplasm both by passive and active transport. However, all cultured cells and resting monocytes contain the vast majority of HMGB1 in the nucleus, indicating that in baseline conditions import is much more effective than export. Cells might transport HMGB1 from the nucleus by acetylating lysine residues which are abundant in HMGB1, thereby neutralizing their basic charge and rendering them unable to function as nuclear localization signals. Nuclear HMGB1 hyperacetylation determines the relocation of this protein from the nucleus to the cytoplasm (in the fibroblasts, for example) or its accumulation into secretory endolysosomes (in activated monocytes and macrophages, for example) and subsequent redirection towards release through a non-classical vesicle-mediated secretory pathway. HMGB1 secretion by already activated monocytes is then triggered by bioactive lysophosphatidylcholine (LPC), which is generated later in the inflammation site from phosphatidylcholine through the action of the secretory phospholipase sPLA2, produced by monocytes several hours after activation. Therefore, secretion of HMGB1 seems to be induced by two signals (Bonaldi et al., 2003) and to take place through three steps: 1) at first, an inflammatory signal promotes HMGB1 acetylation and its relocation from the nucleus to the cytoplasm (step 1) and storage into cytoplasmic secretory vesicles (step 2); then, a secretion signal (extracellular ATP or lysophosphatidylcholine) promotes exocytosis (third step) (Andersson et al., 2002; Scaffidi et al. 2002; Bonaldi et al., 2003; Friedman et al., 2003; Gardella et al., 2002).

Released HMGB1 has been identified as one of the ligands binding to the RAGE receptor. This receptor is expressed in most cell types, and at a high level mainly in endothelial cells, in vascular smooth muscle cells, in monocytes and monophages and in mononuclear phagocytes. Recognition involves the C-terminal of HMGB1. The interaction of HMGB1 and RAGE triggers a sustained period of cellular activation mediated by RAGE up-regulation and receptor-dependent signaling. In particular, the interaction of HMGB1 and RAGE activates several intracellular signal transduction pathways, including mitogen-activated protein kinases (MAPKs), Cdc-42, p21 ras, Rac and the nuclear translocation factor κB (NF-κB), the transcription factor classically linked to inflammatory processes (Schmidt et al., 2001).

According to several experimental evidences, released HMGB1 may also interact with the receptors belonging to the family of the Toll-like receptors (TLR), e.g. with the subclasses TLR2, TLR4, TLR7, TLR8 or/and TLR9.

Furthermore, HMGB1 may also interact with the functional N-terminal lectin-like domain (D1) of thrombomodulin. Due to the ability of the functional D1 domain of thrombomodulin to intercept and bind circulating HMGB1, the interaction of the HMGB1 with the RAGE-receptors and the Toll-like receptors is prevented.

When released *in vivo*, HMGB1 is an extremely potent cytokine and a potent macrophage-stimulating factor. In fact, like other cytokine mediators of endotoxemia, HMGB1 activates *in vitro* a cascade of multiple pro-inflammatory cytokines (TNF, IL-1α, IL-1β, IL-1Ra, IL-6, IL-8, MIP-1α and MIP-1β) from human macrophages. Therefore, HMGB1 behaves as a late mediator during acute inflammation and participates in an important way in the pathogenesis of systemic inflammation, after the early mediator response has been resolved.

The observed pro-inflammatory effects of HMGB1 *in vitro* and the correlation between circulating HMGB1 levels and the development of the pathogenic sequence of systemic inflammation *in vivo* indicate that therapeutically targeting this cytokine-like molecule should be of relevant clinical value, suggesting novel therapeutic approaches by a "late" administration of (selective) antagonists/ inhibitors of the extracellular activities of HMGB1.

Therefore, several attempts were performed to block this extracellular HMGB1 chemokine-protein. Several important approaches were addressed to the administration of antibodies against HMGB1, of antibodies to RAGE, of soluble RAGE (sRAGE), of HMGB1 fragments (for example HMGB1 A Box) and of ethyl pyruvate (Czura et a., 2003; Lotze et al., 2003).

The passive immunization of mice with HMGB1-neutralizing antibodies conferred a highly significant, dose-dependent and lasting protection against lethal doses of endotoxin, even when the first doses of antibodies were given after the TNF peak had passed, suggesting that antagonizing HMGB1 activity late in the clinical course may be an effective treatment approach to potentially lethal sepsis.

Another possibility is to administer mono- or oligoclonal antibodies against the HMGB1 B-box, or its 20 amino acid relevant core which signals through RAGE. Furthermore, HMGB1 A-box, one of the two DNA-binding domains in HMGB1, has been identified as a specific antagonist of HMGB1: highly purified recombinant A-box protected mice from lethal experimental sepsis even when initial treatment was delayed for 24 hours after pathology induction, further suggesting that HMGB1 antagonists may be administered successfully in a clinically relevant window wider than that one used for other known cytokines. Structural function analysis of HMGB1-truncated mutants revealed that the A-box domain of HMGB1 competitively displaces the saturable binding of HMGB1 to macrophages, specifically antagonizing HMGB1 activities. As already seen for the protective activity of anti-HMGB1 antibodies, the administration of the A-box rescues mice from sepsis even when treatment initiated as late as 24 hours after surgical induction of sepsis. HMGB1 antagonist or inhibitors selected from the group of antibodies or antibody fragments that bind to an HMGB1 protein, HMGB1 gene antisense sequences and HMGB1 receptor antagonists are known from US 6.468,533, WO 02/074337 and US 2003/0144201.

Moreover, saturation of circulating HMGB1 by the administration of sRAGE leads to the block of its activities mediated by cellular RAGE, result which can be also obtained by inhibiting RAGE itself with the administration of anti-RAGE antibodies.

Finally, a similar protective response late in the course of sepsis has been observed by administering ethyl-pyruvate, a stable lipophilic derivative and relatively non-toxic food additive, that attenuates the systemic inflammation of ischemia/reperfusion tissue injury and lethal hemorrhagic shock. Ethyl-pyruvate inhibited HMGB1 and TNF release in vitro from endotoxin-stimulated murine macrophages, while in vivo protected mice from peritonitis-induced lethal sepsis, again when dosing was begun 24 hours after this pathology was experimentally induced.

The problem underlying the present invention is the provision of novel agents for the treatment of HMGB1-related pathologies (also often referred to herein as HMGB1-associated pathologies). The aim of the present invention is to use the novel agents as selective HMGB1 antagonists/inhibitors, in order to inhibit in patients the broad spectrum of pathological effects induced by the chemokine itself and/or by the cascade of inflammatory cytokines caused by the extracellular release of the HMGB1 protein.

The solution to this problem is provided by the use of synthetic double-stranded nucleic acid or nucleic acid analogue molecules with a bent shape structure, comprising at least one structural bend, capable of binding to the HMGB1 protein, particularly to an extracellular HMGB1 protein, for the manufacture of a medicament for the prevention or treatment of HMGB1-associated pathologies. It is further within the scope of the present invention, that the synthetic double-stranded nucleic acid or nucleic acid analogue molecules with a bent shape structure, are capable of binding to HMGB1 homologous proteins, in particular to the extracellular HMGB1 homologous proteins.

In the context of the present invention, "HMGB1" includes the non-acetylated form or/and the acetylated form of HMGB1. Likewise "HMGB1 homologous proteins" include the non-acetylated form or/and the acetylated form of HMGB1 homologous proteins. Preferred HMGB1 homologous proteins are HMGB2, HMGB3, HMG-1L10, HMG-4L or/and SP100-HMG.

A homologous protein of HMGB1 is defined in the context of the present invention as a protein having an amino acid sequence which has an identity on the amino acid level of at least 60%, preferably of at least 70%, more preferably of at least 80% and even more preferably of at least 90% compared to the amino acid sequence of the HMGB1 protein.

The term "identity" is understood within the context of the present invention as a percentage value which results when one divides the number of identical amino acids of two amino acid sequences which are to be compared by the number of all the amino acids of one of the two sequences.

The capability of binding to HMGB1 is preferably defined by

(i) the spectrum change of the HMGB1 protein and/or of the synthetic double-stranded nucleic acid/nucleic acid analogue molecules spectra, as determined by the measurements of circular dichroism (CD) studies, as shown for example hereinafter in Example 1, and/or

(ii) the capability of inhibiting the proliferation and/or migration of BAEC cells and/or BASMC cells, whereby an $EC_{50}$ value of $\leq$ 100 nM, preferably of $\leq$ 50 nM, more preferably of $\leq$ 25 nM and even more preferably of $\leq$ 15 nM is observed, as shown for example hereinafter in Example 2.

Therefore, in the use of the present invention, the synthetic double-stranded nucleic acid or nucleic acid analogue molecules with a bent shape structure capable of binding to the HMGB1 protein are preferably capable of binding to the non-acetylated or/and to the acetylated form of HMGB1. Further, in the use of the present invention, the synthetic double-stranded nucleic acid or nucleic acid analogue molecules are preferably capable of binding HMGB1 homologous proteins, in particular HMGB2, HMGB3, HMG-1L10, HMG-4L or/and SP100-HMG, including either the non-acetylated or/and the acetylated forms thereof.

Further, in the context of the present invention, HMGB1-associated pathologies include pathologies related to non-acetylated or/and to acetylated form of HMGB1 or pathologies associated with HMGB1 homologous proteins as defined above, including the non-acetylated or/and acetylated forms of HMGB1 homologous proteins, in particular HMGB2, HMGB3, HMG-1L10, HMG-4L or/and SP100-HMG.

An HMGB1-associated pathology is a condition in a patient wherein an increased concentration of the HMGB1 protein and/or of HMGB1 homologous proteins is present in the biological fluids and tissues, compared to the concentration in normal subjects where these HMGB1 proteins are practically undetectable. The HMGB1-associated pathologies and/or the pathologies associated with HMGB1 homologous proteins are pathologies with a strong inflammatory basis or pathologies which result from the stimulation of cytokine such as TNF-alpha, IL-1, IL-6 etc., or pathologies which result from toxic events, such as intoxication, infection, burn, etc.. In particular high concentrations of the HMGB1 protein and homologous proteins have been found and determined in plasma of patients with sepsis, in plasma and synovial fluid of rheumatoid arthritis patients, in brains of Alzheimer's disease patients, in plasma and tissues of melanoma patients, in plasma of systemic lupus erithematosus patients, in atherosclerotic plaques of atherosclerotic patients, etc. The determination and evidence of HMGB1 protein and/or homologous proteins in biological fluids and tissues, may be detected by common diagnostic tools known by the skilled person in the art, including for example detection by ELISA assays etc.

The synthetic double-stranded nucleic acid or nucleic acid analogue molecules capable of binding to the HMGB1 protein, preferably capable of binding to the non-acetylated or/and acetylated form of HMGB1, may be administered to a patient in a therapeutically effective amount in a method of treating a condition in the patient, which condition is a HMGB1-associated pathology, including pathologies associated with HMGB1 homologous proteins, preferably characterized by HMGB1 activation of an inflammatory cytokine cascade. In the method of the present invention, the HMGB1-associated pathology is preferably a pathology associated with the non-acetylated or/and acetylated form of HMGB1 as well as with the non-acetylated or/and acetylated form of HMGB1 homologous proteins, in particular HMGB2, HMGB3, HMG-1L10, HMG-4L or/and SP100-HMG.

Surprisingly, it was found that synthetic short double-stranded nucleic acid or nucleic acid analogue molecules with at least one structural bend could compete or inhibit extracellular disease-associated HMGB1. Preferably, the double-stranded nucleic acid or nucleic acid analogue molecules according to the present invention have base-paired and unpaired portions. More preferably, the nucleic acid or nucleic acid analogue molecules are selected from the group consisting of bent nucleic acid molecules, cruciform nucleic acid molecules or analogues and combinations thereof. In particular, the bent nucleic acid molecules may, according to the present invention, comprise one or more structural bends within the structure of the molecule. Preferably, the nucleic acid or nucleic acid analogue molecules comprise 1-3 structural bends, more preferably one structural bend.

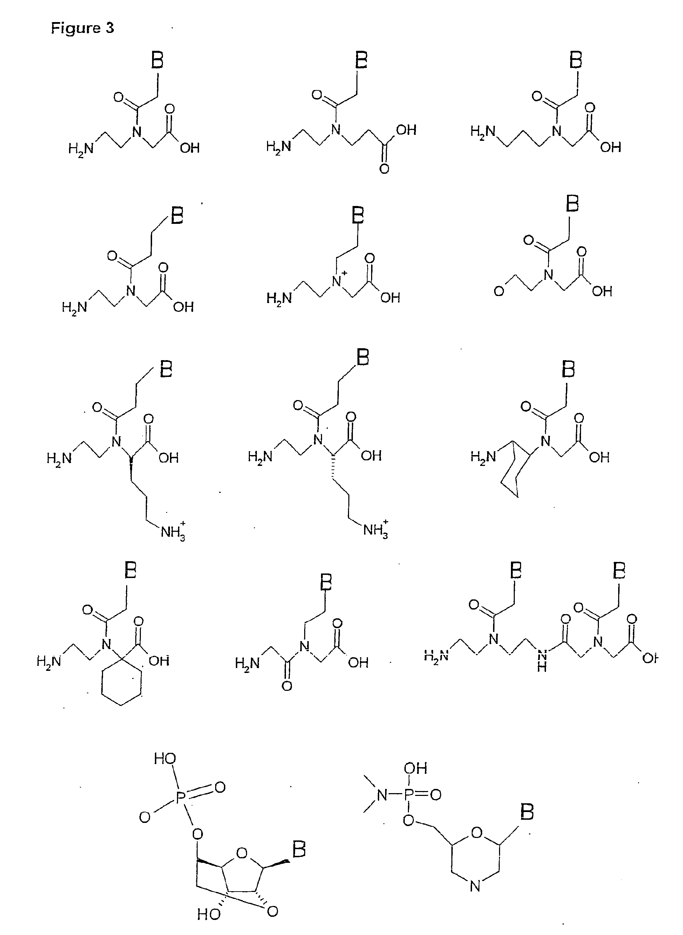

According to the preferred embodiments of the present invention, the nucleic acid molecules are double-stranded DNA molecules, while the nucleic acid analogue molecules are non-naturally occurring building blocks selected from the group consisting of double-stranded PNA molecules, double-stranded LNA molecules, double-stranded DNA/PNA hybrid molecules, double-stranded DNA/LNA hybrid molecules, double-stranded PNA/LNA hybrid molecules, double-stranded DNA/PNA chimera molecules, double-stranded DNA/LNA chimera molecules, and double-stranded PNA/LNA chimera molecules. Moreover, in a further preferred embodiment, the non-naturally occurring building blocks of the present invention are double-stranded nucleic acids containing at least one chemically modified nucleotide building block, selected from sugar-, backbone- and/or nucleobase-modified nucleotide building blocks. Preferred examples of such building blocks are as shown in Figure 3. The LNA modification introduces a 2'-O, 4'-C methylene bridge or a 2'-O, 5'-C methylene bridge in the sugar moiety of a nucleotide or nucleotide analogue. In the context of the present invention, an LNA is a nucleic acid or a nucleic acid analogue, comprising one or more LNA modified nucleotides or LNA modified nucleotide analogues.

The double-stranded nucleic acid or nucleic acid analogue molecule may be conjugated preferably via a terminal position to at least one supplementary moiety. This supplementary moiety may be a targeting moiety, a moiety capable of modifying pharmacokinetic properties etc.. For example, the moiety may be selected from amino acids, peptides, polypeptides, carbohydrates, lipids, hydrophilic or hydrophobic polymers, such as poly (alkylene)glycols, e.g. polyethylene glycol, vitamins or combinations thereof.

The synthetic double-stranded nucleic acid molecule are most preferably bent duplex nucleic acid molecules or analogues or a combination thereof. The bent duplex molecules of the present invention consist of two complementary nucleic acid strands and/or nucleic acid analogue strands. Each strand has a length of from 4 to 80 nucleotide building blocks, preferably from 6 to 40 nucleotide building blocks, most preferably from 8 to 20 nucleotide building blocks. The first complementary strand is preferably 1 to 10 nucleotide building blocks longer than the second complementary strand, forming at least one extruding loop of unpaired nucleotide or nucleotide analogue building blocks.

According to the present invention, the bent nucleic acid molecules or bent nucleic acid analogue molecules used for binding the HMGB1 protein, particularly the extracellular HMGB1 protein, are preferably represented by the general formula I:

$$3'\text{-}(W)_s\text{-}Y_1\ldots Y_n \quad \overset{\displaystyle \ulcorner Z_1 - Z_r \urcorner}{} \quad Y'_1\ldots Y'_m\text{-}(V)_s\text{-}5'$$

(I)

$$5'\text{-}(W')_s\text{-}X_1\ldots X_{n'} \; \text{------} \; X'_1\ldots X'_{m'}\text{-}(V')_s\text{-}3'$$

wherein W, W', X, X', Y, Y', V, V' and Z are independently selected from nucleotide building blocks or nucleotide analogue building blocks. In Formula I, X and Y, X' and Y', W and W' and V and V' respectively are each complementary building blocks, preferably being matched according to the Watson and Crick base pairing. $Z_1 - Z_r$ represent the extruding loop defining the angle of the bent DNA molecule and consisting of unpaired nucleotide building blocks and/or unpaired nucleotide analogue building blocks. n, m, n', and m' are integers from 2 to 20, preferably from 3 to 10; r is an integer from 1 to 10 (or can be 1, 2, 3, 4, 5, 6, 7, 8, 9 or 10), preferably from 2 to 8 or from 4 to 8, and more preferably r = 2, 3, 4, 5 or 6; most preferred are r = 2 or r = 6; and s and s' are in each case independently an integer from 0 to 10, preferably from 0 to 5.

At the terminal positions of the compound of formula (I), nucleotide building blocks or nucleotide analogue building blocks may form a bridged structure which may prevent or reduce degradation, e.g. enzymatic degradation upon administration to a subject in need thereof.

At the terminal positions of the compound of formula (I), one or more supplementary moieties as indicated above, e.g. amino acids, peptides, hydrophilic or hydrophobic polymers etc. and combinations thereof, can be added to the nucleotide building blocks or to the nucleotide analogue building blocks. Depending on the specific supplementary moiety, this may improve the solubility and the pharmacokinetic profile as well as reduce/abrogate immunogenicity of the compound of formula (I).

W, W', X, X', Y, Y', V, V' and Z are preferably DNA building blocks, PNA building blocks, LNA building blocks or sugar and/or backbone-modified nucleic acid building blocks.

Preferably, $Z_1 - Z_r$ at least partially represent nucleotide building blocks with an adenine base, more preferably each $Z_1 - Z_r$ is an adenine nucleotide building block. In an especially preferred embodiment, the loop on the one complementary strand of the bent nucleic acid molecule structure of the present invention is a two adenine loop or a six adenine loop which extrudes from the double helix.

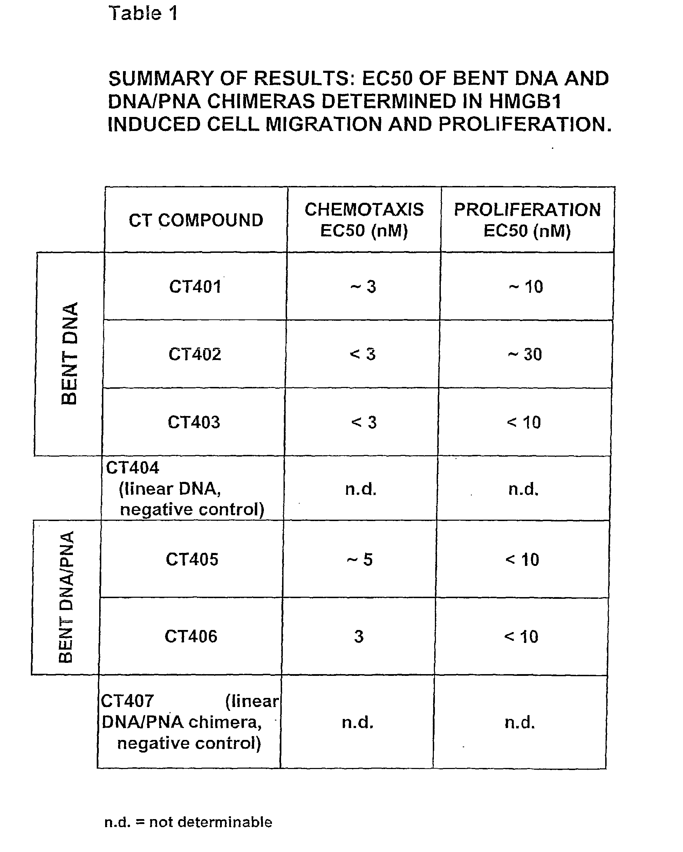

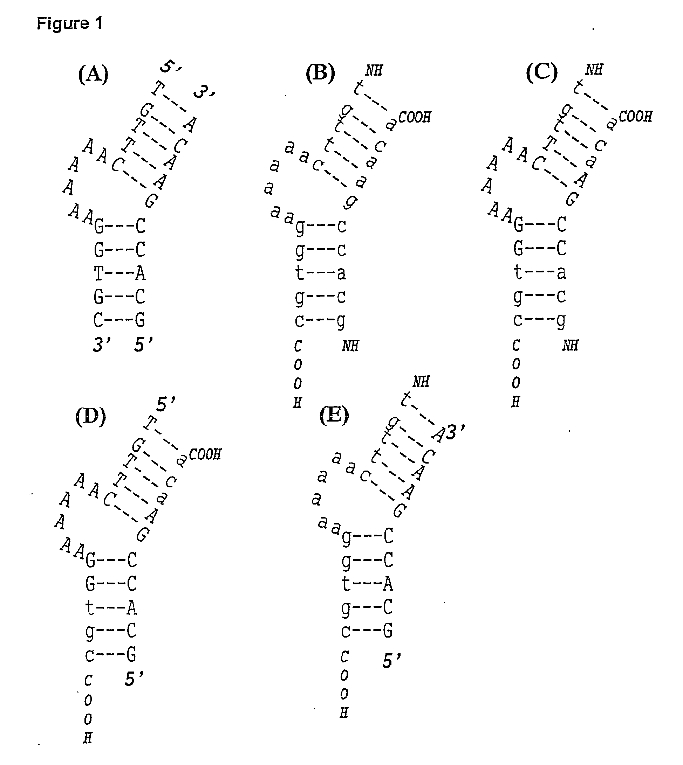

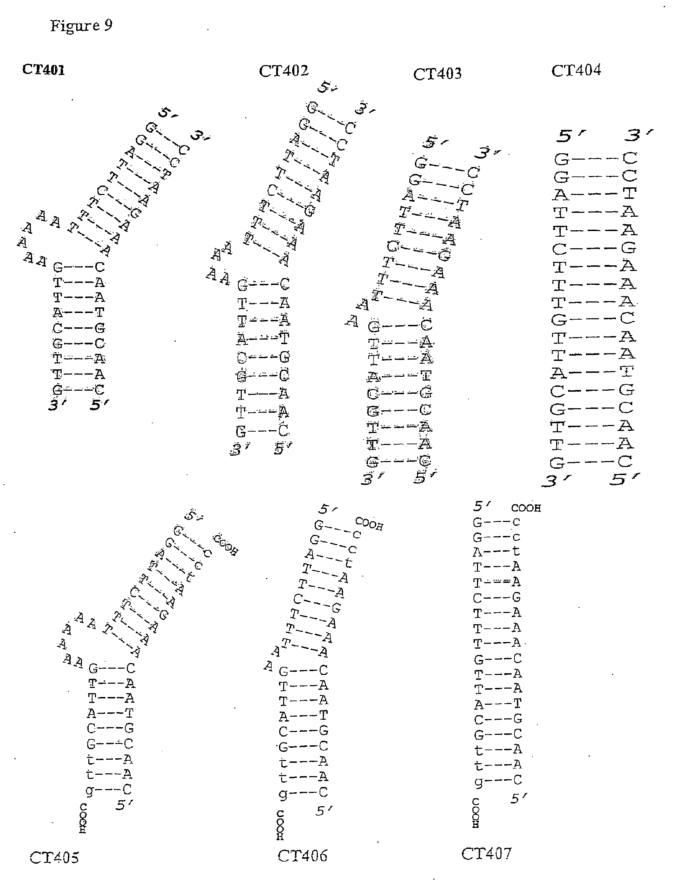

In a further especially preferred embodiment of the present invention, the bent nucleic acid analogue molecule consists of two chimera strands consisting of 16-mer and 10-mer sequences, respectively, wherein on the 3' end of each strand the last three nucleotides are PNA building blocks. Therefore, a preferred nucleic acid molecule is represented by the general formula (I), wherein W', X, X', Y, Y' and V are DNA nucleotide building blocks or/and LNA nucleotide building blocks, Z is an adenine nucleotide or an adenine LNA modified nucleotide, W and V' are PNA nucleotide building blocks, n and m' are 2, n' and m are 5, r is 2 or 6, s is 0 and s' is 3 (as shown in Figure 1D with r = 6 and Figure 9 with r = 2, CT406).

Preferred examples of bent nucleic acid molecules or nucleic acid analogue molecules according to the present invention are shown in Figures 1 and 9.

In a specific example, the compound of formula (I) is represented by a bent duplex nucleic acid molecule (CT401) consisting of two DNA strands of the sequences:

| | |
|---|---|
| 24-mer | 3'OH-GTTGCATTGAAAAAATTTCTTAGG-5'OH |
| 18-mer | 5'OH-CAACGTAAC------AA.AGAATCC-3'OH |

In another specific example, the compound of formula (I) is represented by a bent duplex nucleic acid molecule (CT402) consisting of two DNA strands of the sequences:

| | |
|---|---|
| 22-mer | 3'OH-GTTGCATTGAAAATTTCTTAGG-5'OH |
| 18-mer | 5'OH-CAACGTAAC----AAAGAATCC-3'OH |

In yet another specific example, the compound of formula (I) is represented by a bent duplex nucleic acid molecule (CT403) consisting of two DNA strands of the sequences:

| | |
|---|---|
| 20-mer | 3'OH-GTTGCATTGAATTTCTTAGG-5'OH |
| 18-mer | 5'OH-CAACGTAAC--AAAGAATCC-3'OH |

In a further specific example, the compound of formula (I) is represented by a bent duplex nucleic acid molecule consisting of a DNA/PNA chimeric duplex (CT405). The capital letters represent nucleotide building blocks while the lower case letters represent PNA building blocks:

| | |
|---|---|
| 24-mer | COOH-gttGCATTGAAAAAATTTCTTAGG-5'OH |
| 18-mer | 5'OH-CAACGTAAC------AAAGAAtcc-COOH |

In yet another specific example, the compound of formula (I) is represented by a bent duplex nucleic acid molecule consisting of a DNA/PNA chimeric duplex (CT406). The capital letters represent nucleotide building blocks while the lower case letters represent PNA building blocks:

| | |
|---|---|
| 20-mer | COOH-gttGCATTGAATTTCTTAGG-5'OH |

18-mer        5'OH-CAACGTAAC--AAAGAAtcc-COOH

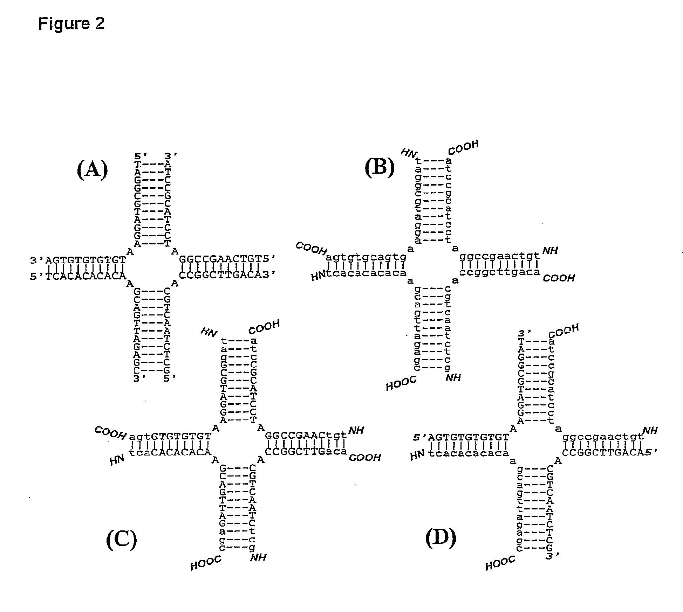

According to the present invention, the cruciform nucleic acid molecule or cruciform nucleic acid analogue molecule which binds to the HMGB1 protein, particularly the extracellular HMGB1 protein, consists of four nucleic acid strands and/or nucleic acid analogue strands. These four strands have complementary hybridising portions and, between the hybridising portions, the single strands comprise 0 to 4, preferably 0 to 1, unpaired nucleotide building blocks or nucleotide analogue building blocks. The four strands pair to form a cruciform structure molecule. Preferred examples of cruciform molecules according to the present invention are shown in Figure 2. In an especially preferred embodiment of the present invention, the cruciform structure comprises at least one non-naturally occurring nucleotide building block, e.g. a cruciform chimera structure, in which each of the four strands is a DNA sequence strand, wherein, on the 3' end and/or the 5' end of each strand, at least one, preferably two or three, nucleotides are non-naturally occurring, e.g. PNA building blocks.

In a specific example of the cruciform nucleic acid molecule used in the present invention for binding the HMGB1 protein is represented by a cruciform DNA molecule (CT400), consisting of four DNA strands of the sequences as shown below:

```
                              o13
                              CAG CTG
                              GCC CAG CTG
                              TCC GGA GGG CGG
                              CC GG
 oll AGC GCT CTC ACA CGG G      G CGG ACG TTA ACC CC
     CG CGA GAG TGT GCC C  GG  C GCC TGT AAT CCG GG  allmut.dir
                              CC TAA
                              AGA TCT
                              ACC GGT
                              TCT AGA
                              o14
```

    oll: AGC GCT CTC ACA CGG GCC TCC GCC CAG CTG

    ol3: CAG CTG GGC GGA GGG CGG ACG TTA ACC CC

    allmut.dir: GG GGT TAA CGT CCG CGG TAA TCT GGT AGA

    ol4: TCT ACC AGA TTA CCC CCG TGT GAG AGC GC

Surprisingly, the double-stranded nucleic acid molecules and nucleic acid analogue molecules of the present invention exhibit a high capability for binding the HMGB1 protein and/or to HMGB1 homologous proteins.

Therefore, the invention is directed to the use of the above-mentioned nucleic acid compounds for the prevention or treatment of extracellular HMGB1-related pathologies which are mediated by an inflammatory cytokine cascade.

Non limiting examples of conditions which can be usefully treated using the present invention include the broad spectrum of pathological conditions induced by the HMGB1-chemokine and by the HMGB1-induced cascade of inflammatory cytokines grouped in the following categories: inflammatory disease, autoimmune disease, systemic inflammatory response syndrome, reperfusion injury after organ transplantation, cardiovascular affections, obstetric and gynecologic disease, infectious disease, allergic and atopic disease, solid and liquid tumor pathologies, transplant rejection diseases, congenital diseases, dermatological diseases, neurological diseases, cachexia, renal diseases, iatrogenic intoxication conditions, metabolic and iodiopathic diseases, and ophthalmological disease.

In particular, the pathologies belonging to inflammatory and autoimmune diseases include rheumatoid arthritis/seronegative arthropathies, osteoarthritis, inflammatory bowel disease, Crohn's disease, systemic lupus erythematosus, iridoeyelitis/uveitis, optic neuritis, idiopathic pulmonary fibrosis, systemic vasculitis/Wegener's granulomatosis, sarcoidosis, orchitis/vasectomy reversal procedures. Systematic inflammatory response includes sepsis syndrome (including gram positive sepsis, gram negative sepsis, culture negative sepsis, fungal sepsis, neutropenic fever, urosepsis, septic conjunctivitis), meningococcemia, trauma hemorrhage, hums, ionizing radiation exposure, acute and chronic pancreatitis, adult respiratory distress syndrome (ARDS), prostatitis. Reperfusion injury includes post-pump syndrome and ischemia-reperfusion injury. Cardiovascular disease includes atherosclerosis, intestinal infarction, cardiac stun syndrome, myocardial infarction, congestive heart failure and restenosis. Obstetric and gynecologic diseases include premature labour, endometriosis, miscarriage and infertility. Infectious diseases include HIV infection/HIV neuropathy, septic meningitis, hepatitis B and C virus infection, herpes virus infection, septic arthritis, peritonitis, pneumonia epiglottitis, E. coli 0157: H7, haemolytic uremic syndrome/thrombolytic thrombocytopenic purpura, malaria, Dengue hemorrhagic fever, leishmaniasis, leprosy, toxic shock syndrome, streptococcal myositis, gas gangrene, mycobacterium tuberculosis, mycobacterium avium intracellulare, pneumocystis carinii pneumonia, pelvic inflammatory disease, orchitis/epidydimitis, legionella, Lyme disease, influenza A, Epstein-Barr Virus, Cytomegalovirus, viral associated hemiaphagocytic syndrome, viral encephalitis/aseptic meningitis. Allergic and atopic disease include asthma, allergic rhinitis, eczema, allergic contact dermatitis, allergic conjunctivitis, hypersensitivity pneumonitis. Malignancies (solid and liquid tumor pathologies) include neoplastic diseases, melanoma, ALL, AML, CML, CLL, Hodgkin's disease, non Hodgkin's lymphoma, Kaposi's sarcoma, colorectal carcinoma, nasopharyngeal carcinoma, malignant histiocytosis and paraneoplastic syndrome/hypercalcemia of malignancy. Transplant diseases include organ transplant rejection and graft-versus-host disease. Congenital disease includes cystic fibrosis, familial hematophagocytic lymphohistiocytosis and sickle cell anemia. Dermatologic disease includes psoriasis, psoriatic arthritis and alopecia. Neurologic disease includes neurodegenerative diseases, Alzheimer's Disease, Parkinson's Disease, multiple sclerosis, amyotrophic lateral sclerosis, migraine headache, amyloid-associated pathologies, prion diseases/Creutzfeld-Jacob disease, cerebral infarction and peripheral neuropathies. Renal disease includes nephrotic syndrome, hemodialysis and uremia. Iatrogenic intoxication condition includes OKT3 therapy, Anti-CD3 therapy, Cytokine therapy, Chemotherapy, Radiation therapy and chronic salicylate intoxication. Metabolic and idiopathic disease includes Wilson's disease, hemachromatosis, alpha-1 antitrypsin deficiency, diabetes, Hashimoto's thyroiditis, osteoporosis, hypothalamic-pituitary-adrenal axis evaluation and primary biliary cirrhosis. Ophthalmological disease includes glaucoma, retinopathies and dry eye.

Further, pathologies which can be usefully treated using the present invention include further multiple organ dysfunction syndrome, muscular dystrophy, septic meningitis, atherosclerosis, appendicitis, peptic, gastric or duodenal ulcers, ulcerative pseudomembranous, acute or ischemic colitis, diverticulitis, epiglottis, achalasia, cholangitis, cholecystitis, enteritis, Whipple's disease, asthma, allergy, allergic rhinitis, anaphylactic shock, immune complex disease, organ necrosis, hay fever, septicaemia, endotoxic shock, hyperpyrexia, eosinophilic granuloma, granulomatosis, sarcoidosis, septic abortion, vaginitis, prostatitis, urethritis, emphysema, rhinitis, alvealitis, bronchiolitis, pharyngitis, pneumoultramicroscopicsilicovolcanoconiosis, pleurisy, sinusitis, influenza, respiratory syncytial virus infection, disseminated bacteremia, candidiasis, filariasis, amebiasis, hydatid cyst, dermatomyositis, burns, sunburn, urticaria, warts, wheal, vasulitis, angiitis, endocarditis, pericarditis, myocarditis, arteritis, thrombophlebitis, periarteritis nodosa, rheumatic fever, celiac disease, encephalitis, cerebral embolism, Guillaume-Barre syndrome, neuritis, neuralgia, spinal cord injury, paralysis, uveitis, arthriditis, arthralgias, osteomyelitis, fasciitis, Paget's disease, gout, periodontal disease, synovitis, myasthenia gravis, Goodpasture's syndrome, Babcet's syndrome, ankylosing spondylitis, Barger's disease, Retier's syndrome, bullous dermatitis (bullous pemphigoid), pemphigous and pemphigous vulgaris, necrotizing enterocolitis.

In a further aspect of the invention the use of the nucleic acid or nucleic acid analogue molecules described above is in combination with at least one further agent capable of inhibiting an early mediator of the inflammatory cytokine cascade. Preferably, this further agent is an antagonist or inhibitor of a cytokine selected from the group consisting of TNF, IL-1$\alpha$, IL-1$\beta$, IL-Ra, IL-8, MIP-1$\alpha$, MIF-1$\beta$, MIP-2, MIF and IL-6. For example, the further agent used in combination with the nucleic acid molecules of the present invention is preferably an antibody to RAGE, a nucleic acid or nucleic acid analogue capable of inhibiting RAGE expression, e.g. an antisense molecule, a ribozyme or a RNA interference molecule, or a small synthetic molecule antagonist of the HMGB1 interaction with RAGE or a soluble RAGE (sRAGE). The small synthetic molecule antagonist of the HMGB1 interaction with RAGE preferably has a molecular weight of less than 1000 Dalton. The small synthetic molecule antagonist preferably inhibits the interaction of RAGE with the non-acetylated form or/and with the acetylated form of HMGB1, and with the non-acetylated form or/and with the acetylated form of HMGB1 homologous proteins, in particular HMGB2, HMGB3, HMG-1L10, HMG-4L or/and SP100-HMG

In another embodiment, the further agent used in combination with the nucleic acid or nucleic acid analogue of the present invention is an inhibitor of the interaction of a Toll-like (TLR) receptor, e.g. of TLR2, TLR4, TLR7, TLR8 or/and TLR9, with HMGB1, which inhibitor may be, for example, a monoclonal or polyclonal antibody, a nucleic acid or nucleic acid analogue capable of inhibiting TLR expression, e.g. an antisense molecule, a ribozyme or a RNA interference molecule, or a synthetic molecule preferably having a size of less than 1000 Dalton. The inhibitor may be a known inhibitor of a Toll-like receptor (TLR), e.g. of TLR2, TLR4, TLR7, TLR8 or/and TLR9. The inhibitor preferably inhibits the interaction of the Toll-like receptor with the non-acetylated form or/and the acetylated form of HMGB1 and with the non-acetylated form or/and with the acetylated form of HMGB1 homologous proteins, in particular HMGB2, HMGB3, HMG-1L10, HMG-4L or/and SP100-HMG.

In still another embodiment, the further agent used in combination with the nucleic acid or nucleic acid analogue of the present invention is the functional N-terminal lectin-like domain (D1) of thrombomodulin. The D1 domain of thrombomodulin is able to intercept the non-acetylated form and/or the acetylated form of released HMGB1 and of released HMGB1 homologous proteins, in particular HMGB2, HMGB3, HMG-1L10, HMG-4L or/and SP100-HMG, preventing thus their interaction with RAGE and Toll-like receptors. The D1 domain of thrombomodulin may be native or mutated in order to make it resistant to proteases.

The double-stranded nucleic acid or nucleic acid analogue molecules of the present invention are usually administered as a pharmaceutical composition. The administration may be carried out by known methods, e.g. by injection, in particular by intravenous, intramuscular, subcutaneous or intraperitoneal injection or by infusion, by oral, topical, nasal, inhalation, aerosol or rectal application, etc. The administration may be local or systemic.

Therefore, the invention also relates to a pharmaceutical composition containing as an active agent at least one of the double-stranded nucleic acid or nucleic acid analogue molecules as described above, together with a pharmaceutical carrier. The composition may be used for diagnostic or for therapeutic applications. For diagnostic or therapeutic applications, the composition may be in the form of a solution, e.g. an injectable solution, a cream, ointment, tablet, suspension or the like. The carrier may be any suitable pharmaceutical carrier.

The at least one of the double-stranded nucleic acid or nucleic acid analogue molecules in the pharmaceutical composition of the present invention is preferably capable of binding to the HMGB1 protein, more preferably capable of binding to the non-acetylated or/and acetylated form of HMGB1.

The pharmaceutical composition of the present invention may also comprise pharmaceutically acceptable salts of the at least one double-stranded nucleic acid or nucleic acid analogue molecule, including salts of inorganic acids, such as hydrochloric acid, hydrobromic acid, hydriodic acid, sulfuric acid, phosphoric acid, metaphosphoric acid, nitric acid and the like, and salts of organic acids, such as acetic acid, citric acid, tartaric acid, malic acid, malonic acid, benzoic acid, sulfonic acid and the like. Furthermore, the at least one double-stranded nucleic acid or nucleic acid analogue molecule may form pharmaceutically acceptable salts with a pharmaceutically acceptable cation, including alkali cations (e.g. lithium, sodium, or/and potassium cations), earth alkali cations (e.g. magnesium, calcium or/and barium cations), the ammonium cation and organic cations, such as ammonium cations substituted by aliphatic and aromatic residues. Further, amino acids may form salts with the at least one double-stranded nucleic acid or nucleic acid analogue molecule. The person skilled in the art knows suitable pharmaceutically acceptable salts for preparation of the pharmaceutical composition of the present invention.

The pharmaceutical composition of the present invention may further comprise as excipients common auxiliary substances known by a person skilled in the art, including macromolecular substances (e.g. polyethyleneglycole (PEG)), starch, gelatin, pectin, cellulose, methylcellulose, hydroxypropylcellulose, ethylcellulose, polyvinylpyrrolidone, polyvinyl alcohol, lipids, amino acids, cyclodextrins), sugars and sugar alcohols (e.g. lactose, saccharose, glucose, mannitol, sorbitol), emulsifiers, diluents and the like.

The present invention further relates to a kit for determining HMGB1 in body fluids, preferably serum or/and plasma, obtained from a patient suspected to be affected by an HMGB1-associated pathology, preferably by an inflammatory, cardiovascular, neurodegenerative, neoplastic or/and autoimmune pathology, comprising at least one double-stranded nucleic acid or nucleic acid analogue molecules as described above. The kit may also comprise the pharmaceutical composition as described above.

The kit of the present invention preferably relates to the determination of the non-acetylated or/and the acetylated form of HMGB1. Further, the kit of the present invention may relate to the determination of the non-acetylated or/and acetylated form of HMGB1 homologous proteins, in particular HMGB2, HMGB3, HMG-1L10, HMG-4L or/and SP100-HMG.

Further, the double-stranded nucleic acid or nucleic acid analogue molecules of the present invention can be reversibly immobilised on the surface of a medical device. The medical device can thus be reversibly loaded with the molecules of the present invention, in particular by binding, embedding and/or absorbing the nucleic acid or nucleic acid analogue molecules onto the surface of the medical device or on a coating layer on the surface of the medical device. After contacting the medical device with body fluid, the reversibly immobilised compounds are liberated. Consequently, the described medical devices act as drug delivery devices eluting the molecules of the invention, whereby the drug delivery kinetics can be controlled, providing a controlled or sustained drug delivery, for example. The coating technologies of medical devices are well known to the person skilled in the art.

Therefore, a further aspect of the present invention is the use of the synthetic double-stranded nucleic acid or nucleic acid analogue molecules of the present invention, wherein said molecules are reversibly immobilised on the surface of medical devices. Preferably, said medical devices are surgical instruments, implants, catheters or stents, e.g. stents for angioplasty. Most preferably, the medical device according to the invention is a drug-eluting stent (DES).

A still further aspect of the present invention is a medical device reversibly coated with double- stranded nucleic acid or nucleic acid molecules according to the present invention.

Further, the present invention is explained in more detail in the following Figures and Examples.

**Figure and Table Legends**

Table 1 - Summary of the results of the example: $EC_{50}$ of bent DNA and DNA/PNA chimeras determined in HMGB1-induced cell migration and proliferation.

Figure 1 - Examples of structures of bent nucleic acid molecules or bent nucleic acid analogue molecules, according to the present invention. The black capital letters represent the natural oligonucleotide sequence while the red letters represent the PNA base sequence. (A): Bent DNA duplex; (B): Bent PNA duplex; (C) and (D): Bent DNA/PNA chimera duplex; (E): Bent DNA/PNA hybrid duplex.

Figure 2 - Examples of structures of cruciform nucleic acid molecules or cruciform nucleic acid analogue molecules according to the present invention. The black capital letters represent the natural oligonucleotide sequence, while the red letters represent the PNA sequence. (A): cruciform DNA, (B): cruciform PNA, (C): cruciform DNA/PNA chimera, (D): cruciform DNA/PNA hybrid.

Figure 3 - Examples of non-naturally occurring chemically modified nucleotide building blocks, wherein the letter "B" denotes a nucleotidic base.

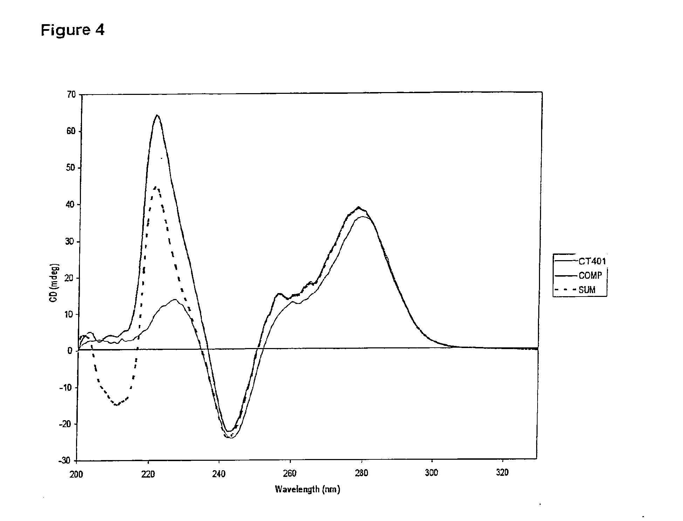

Figure 4 - Circular dichroism spectra of 1) the bent duplex DNA with a polyadenylic loop protruding from the duplex (CT401 dark blue), 2) the HMGB1-bent DNA (CT 401) complex (COMP black) and 3) the normalized sum of the spectra of HMGB1 and bent duplex DNA (CT 401) (SUM dashed black).

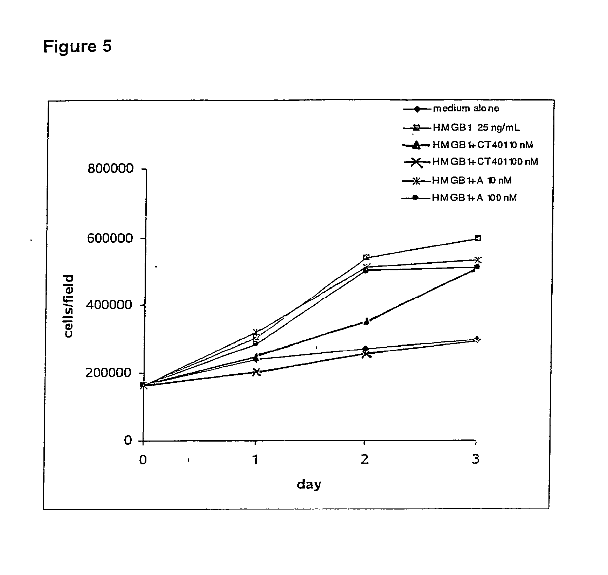

Figure 5 - Graphic representation of the results of the proliferation assay performed with the bent DNA CT401 of the present invention. The bent DNA CT401 concentration-dependently inhibits/antagonizes the proliferation of BAEC cells induced by HMGB1, while control linear duplex DNA (A) does not.

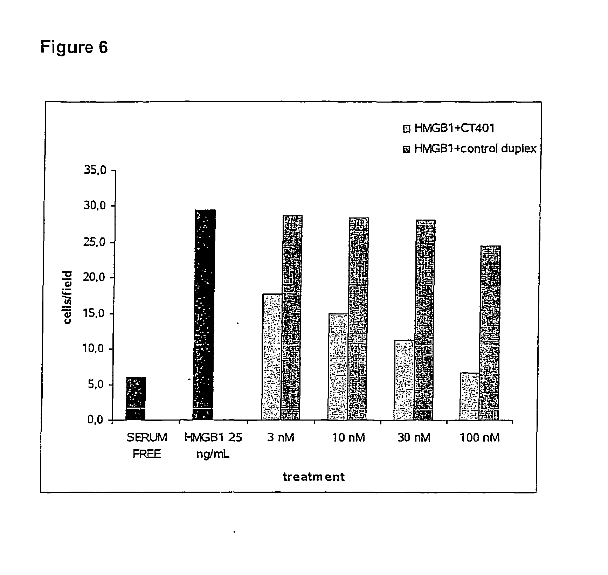

Figure 6 - Graphic representation of the results of the chemotaxis assay performed with the bent DNA CT401 of the present invention. The bent DNA CT401 concentration-dependently inhibits/antagonizes the migration of BAEC cells induced by HMGB1 already in a nM range, while control duplex DNA does not.

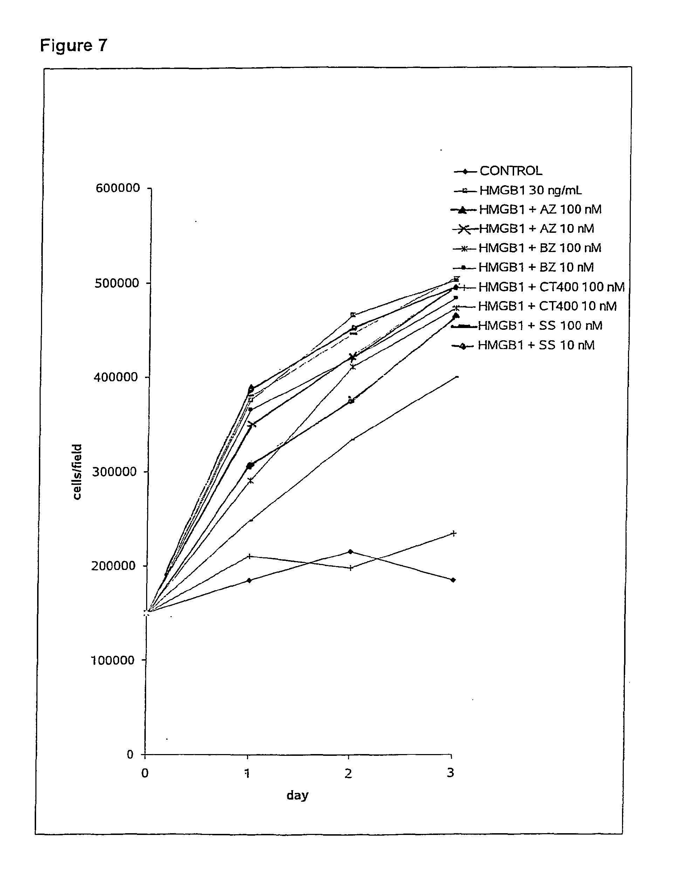

Figure 7 - Graphic representation of the results of the proliferation assay performed with the cruciform DNA CT400 of the present invention. The cruciform DNA CT400 concentration-dependently inhibits/antagonizes the proliferation of BAEC cells induced by HMGB1, while control duplex DNAs' (AZ and BZ) and single stranded DNA (SS) do not.

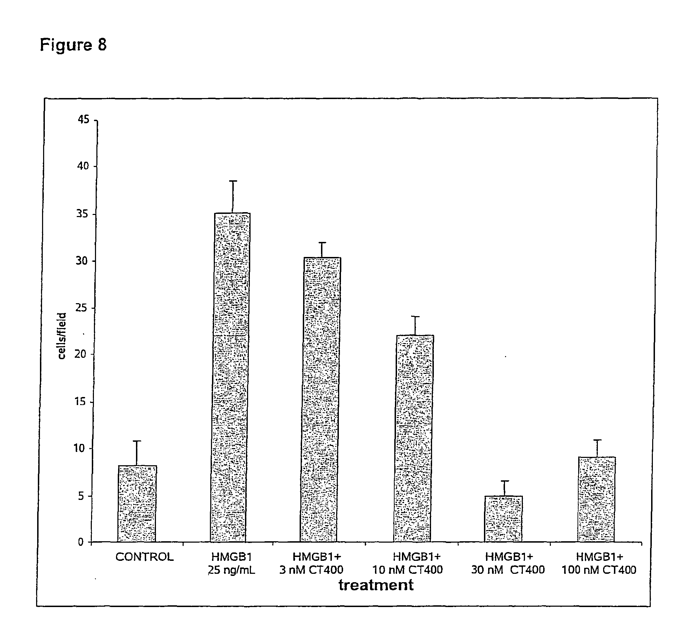

Figure 8 - Graphic representation of the results of the chemotaxis assay performed with the cruciform DNA CT400 of the present invention. The cruciform DNA CT400 concentration-dependently inhibits/antagonizes the migration of BAEC cells induced by HMGB1 and its activity is already present in a nM range.

Figure 9 - Structures of bent nucleic acid molecules CT401, CT402 and CT403 or bent molecules CT405 and CT406 comprising nucleic acid analogues. The linear duplexes CT404 and CT407 serve as controls. The capital letters represent nucleotide building blocks while the lower case letters represent PNA building blocks.

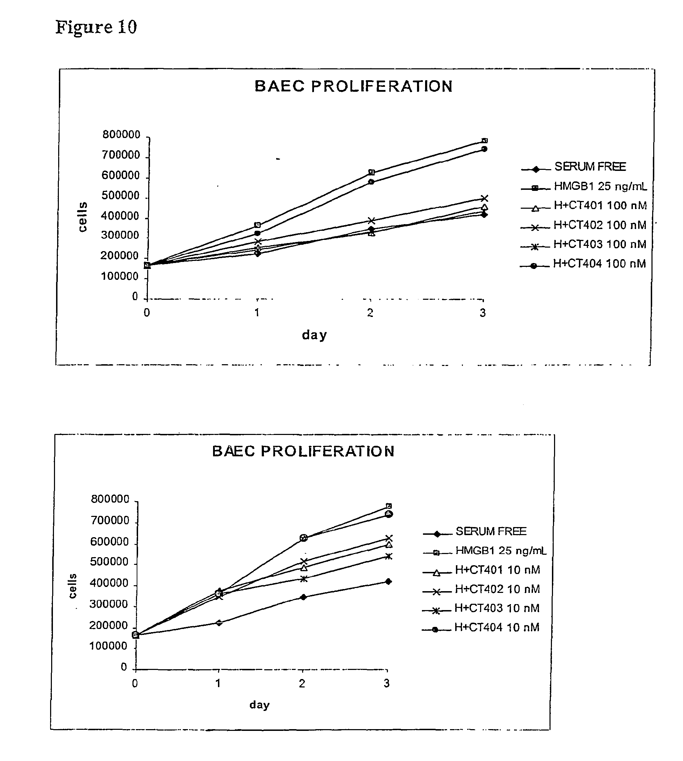

Figure 10 - Graphic representation of the results of a proliferation assay performed with the bent DNA molecules CT401, CT402, and CT403 of the present invention and control DNA CT404 without bending. The bent DNA molecules CT401, CT402, and CT403 concentration-dependently inhibits/antagonizes the proliferation of BAEC cells induced by HMGB1, while control linear duplex DNA CT404 does not.

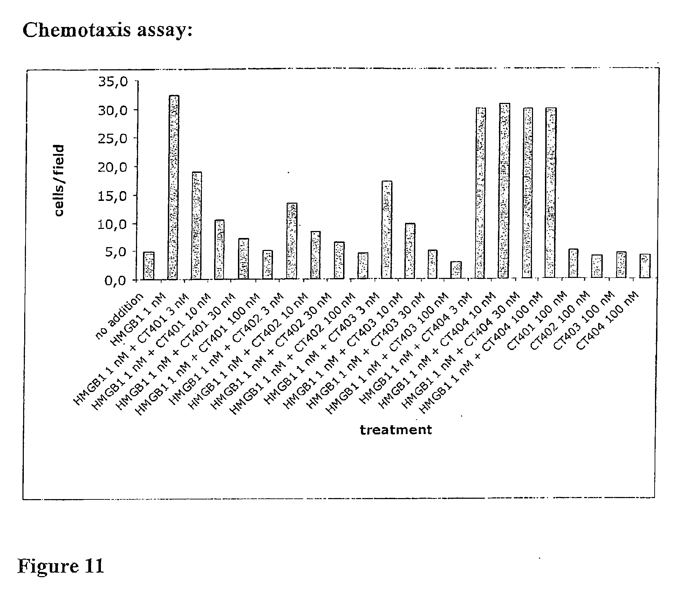

Figure 11 - Graphic representation of the results of a chemotaxis assay performed with the bent DNA molecules CT401, CT402, and CT403 of the present invention and control DNA CT404 without bending. The bent DNA molecules CT401, CT402, and CT403 concentration-dependently inhibits/antagonizes the migration of BAEC cells induced by HMGB1 already in a nM range, while control duplex DNA CT404 does not.

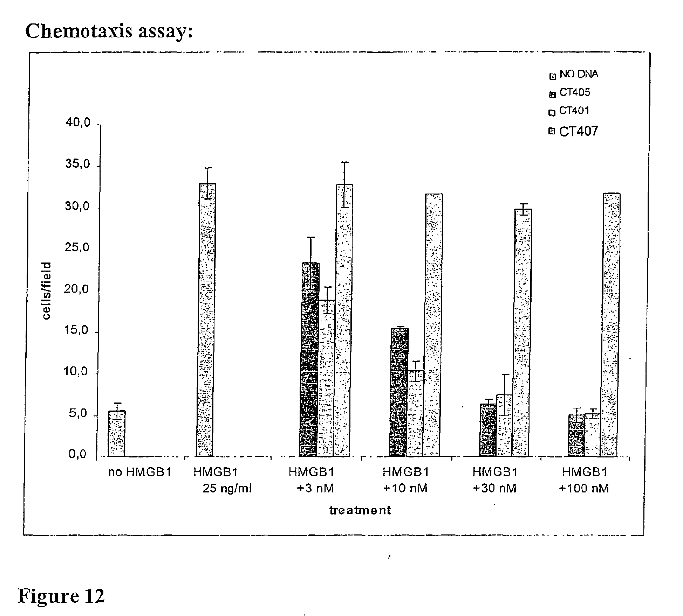

Figure 12 - Graphic representation of the results of a chemotaxis assay performed with the bent DNA/PNA chimeric duplex CT405 and the bent DNA molecule CT401 of the present invention and the chimeric linear duplex CT407 as a control. The bent DNA/PNA chimeric duplex CT405 and the bent DNA molecule CT401 concentration-dependently inhibits/antagonizes the migration of BAEC cells induced by HMGB1 already in a nM range, while control CT407 does not.

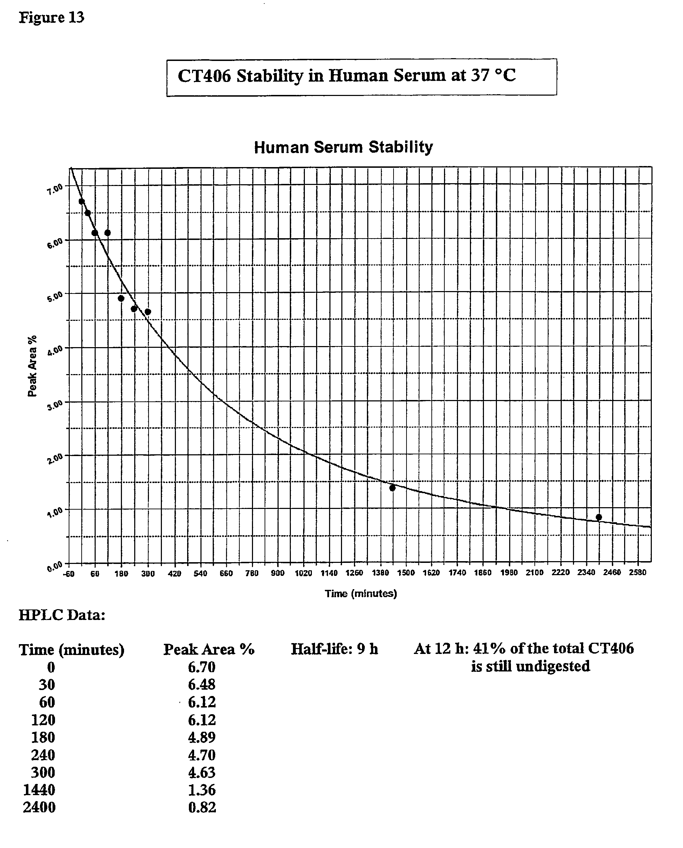

Figure 13 - Graphic representation of the stability of CT406 in human serum at 31°C - CT406 shows a half life time of 9 h. After 12 h incubation time, undiffered CT406 was still 41 % of the total amount.

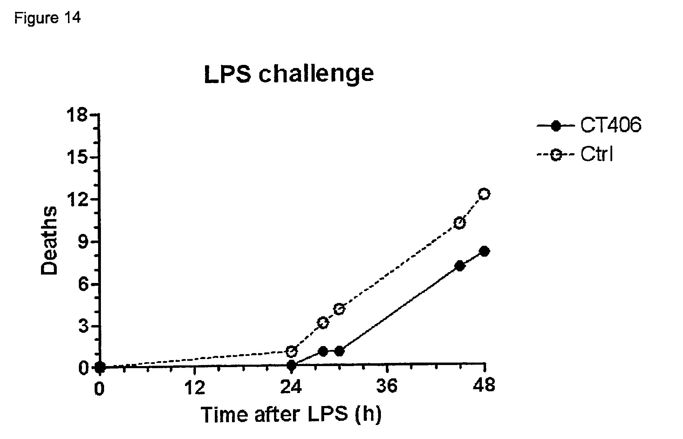

Figure 14 - Graphic representation of the results of LPS-induced endotoxemia trials in mice with the bent DNA molecule CT406.

## Examples

### Example 1

Protein Binding experiments by Circular Dichroism (CD).

To check for protein binding by the different bent and cruciform structures object of the present invention, a CD study was performed. All CD spectra were collected on a Jasco J710 spectropolarimeter equipped with a NesLab RTE111 thermal controller unity, using a quartz cylindrical cuvette with a 1 cm path length (Jasco). We always used a scan speed of 20 nm/min, a bandwidth of 1 nm, a resolution of 1 nm. As an example of the experimental conditions used, we will discuss the case of a bent DNA duplex binding to HMGB1.
In a first experiment, the bent duplex nucleic acid molecule CT 401 described above was used. The duplex was prepared by adding to water an equimolar amount of both strands, heating the resulting solution to 90 °C for five minutes and cooling the solution at room temperature (R.T.). The CD spectrum confirmed the formation of a DNA duplex with an adenine loop extruding from the double helix as expected. A CD spectrum of the protein HMGB1 was recorded, to check for its structural integrity. To this sample, an excess of HMGB1 protein was added and the CD spectrum was recorded again. The spectrum of the complex showed clearly the interaction of the protein, which caused a small unstacking of the DNA bases revealed by a decrease in the 280 nm band of the CD spectrum (possibly a small opening of the double helical parts near to the adenine loop) and a strong change in the structure of the adenine loop revealed by a corresponding change in the 220 nm positive band arising from the adenine loop (possibly caused by the bend adopted by the DNA in presence of the protein). The effects observed in the CD spectrum are most probably due to a strong HMGB1-DNA interaction, since the spectrum of the protein+DNA sample is different from the normalised sum of the spectra of HMGB1 and duplex DNA. Figure 4 shows the circular dichroism spectra obtained, wherein the dark blue line (indicated with CT401 in the legend of Figure 4) represents the spectrum of the DNA duplex compound, the black line (indicated with COMP in the legend of Figure 4) represents the spectrum of the HMGB1-bent DNA complex and the dashed black line (indicated with SUM in the legend of Figure 4) represents the normalised sum of the spectra of HMGB1 protein and duplex DNA compound.

### Example 2

To investigate whether the synthetic molecules belonging to the above structural classes are able to inhibit in vitro the HMGB1 induced proliferation and migration activities of bovine aorta endothelial cells (BAEC), two biological assays, the proliferation and the chemotaxis assays, were performed in the presence or in the absence of CT401 (DNA bent derivative) and of CT400 (DNA cruciform derivative).

Proliferation Assay

The proliferation assay was performed as described by Palumbo et al., (2004).
BAEC cells (bovine aorta endothelial cells) were seeded in 6-well plates ($10^5$ cells/well) and grown in RPMI medium supplemented with 20% FCS. After 24 h, the medium was replaced with serum-free RPMI and cell were then starved for 16 hours to synchronize the cell population. Vehicle (negative control or basal proliferation) or 30 ng/ml (1 nM) of HMGB1 (bacterially made) were added in the presence or in the absence of 10 or 100 nM of the test compounds (dissolved and diluted in serum-free medium). The assay performed with the bent DNA CT401 was also carried out with a control linear oligo DNA (indicated as A in the legend of Figure 5), consisting of an 18mer strand and its complementary strand. Similarly, the assay performed with cruciform DNA CT400 was also carried out with duplex DNA's (indicated with AZ and BZ, respectively, in the legend of Figure 7), and with a single-stranded DNA (indicated as SS in the legend of Figure 7) as control compound. Each experimental point represents the mean +/- standard deviation (SD) of triplicate determinations. The experiment was repeated three times. BAEC cell proliferation was determined by detaching the cells from the plate at the indicated times, and counting the Trypan-blue excluding cells under the microscope. The inhibition of the HMGB1-induced proliferation activity of BAEC cells by the bent DNA compound and the cruciform DNA compound of the present invention are shown in Figures 5 and 7, respectively.

In a further assay according to the protocol as described above, it was demonstrated that the bent molecules CT401, CT402, CT403, CT405 and CT406 concentration-dependently inhibit/antagonize the proliferation of BAEC cells induced by HMGB1. The bent DNA molecules CT401, CT402 and CT403 exhibited an $EC_{50}$ of ~10nM, ~30nM and < 10nM, respectively, while control linear duplex DNA CT404 does not (Figure 10/Table 1). The bent DNA/PNA duplexes CT405 and CT406 showed an $EC_{50}$ of < 10 nM while the control compound CT407 had no effect (see Table 1).

In a further experiment, proliferation of BASMC (bovine aorta smooth muscle cells) in the presence of the specific compounds of the present invention was tested (protocol described in Palumbo et al., 2004). BASMC cells were seeded in 6-well plates ($10^5$ cells/well) and grown in RPMI medium supplemented with 20% FCS. After 24 h, the medium was replaced with serum-free RPMI and cell were then starved for 16 hours to synchronize the cell population. Vehicle (negative control or basal proliferation) or 25 ng/ml (1 nM) of HMGB1 (bacterially made) were added in the presence or in the absence of the compounds of the present invention dissolved as described above. Each experimental point represents the mean +/- SD of triplicate determinations. The experiment was repeated three times. BASMC cell proliferation was determined by detaching the cells from the plate at the indicated times (on days 1, 2, 3, and 4 of culturing) and counting the Trypan-blue excluding cells under the microscope. A concentration dependent inhibition of proliferation of BASMC was observed in the bent molecules of the present invention ($EC_{50}$ < 10 nM). The linear control compounds had no effect.

Chemotaxis Assay

Chemotaxis assays were performed using well-known protocols, in particular as described by Palumbo et al., (2004).

Modified Boyden chambers were used with filters having 5-8 $\mu$m pore size and treated with gelatin type A from porcine skin 5 $\mu$g/ml). BAEC cells (bovine aorta endothelial cells) were resuspended in serum-free DMEM and a sample of 40,000 cells was added to the upper well of a Boyden chamber. The molecules to be tested were dissolved and diluted in the same serum-free medium and added to the lower well of the chamber; HMGB1 (bacterially made) concentration was 1 nM, test compounds were 10-100 nM. The assay performed with bent DNA CT401 was also carried out with a linear duplex DNA as indicated above as control compound. Cell migration was allowed at 37+/-0.5 °C for 4 hours, then cells were scraped off the upper surface, and filters were fixed in ethanol and stained in a solution of modified Giemsa stain (Accustain, Sigma). All experiments were performed at least twice in triplicate. The inhibition of HMGB1-induced migration activity of BAEC cells by the bent DNA compound and the cruciform DNA compound of the present invention are shown in Figures 6 and 8, respectively. Results, as shown in Figures 6 and 8, are the mean +/- SD of the number of cells counted in 10 high-power fields per filter and expressed as folds over control. Random cell migration, i.e. migration in the absence of chemoattractant, was given the arbitrary value of 100%. Statistical analysis was performed using Student's t test for pairwise comparisons of treatment, or an ANOVA model for the evaluation of treatments with increasing concentrations of a reagent.

In a further assay according to the protocol as described above, it was demonstrated that the bent molecules CT401, CT402, CT403, CT405 and CT406 concentration-dependently inhibit/antagonize the migration of BAEC cells induced by HMGB1 already in a nM range, while control duplex DNA CT404 does not. The bent DNA molecule CT401 exhibited an $EC_{50}$ of ~3 nM. The $EC_{50}$ of CT402 and CT403 were < 3 nM (Figure 11/Table 1). Moreover, a concentration-dependent inhibitory/antagonistic effect on the migration of BAEC cells induced by HMGB1 could be observed in the bent DNA/PNA chimeric duplexes CT405 and CT406 of the present invention in the nanomolar range, while the chimeric linear duplex CT407 serving as a control does not show an inhibitory/antagonistic effect (Figure 12). The $EC_{50}$ value of CT405 is -5 nM. The $EC_{50}$ value of CT406 is 3 nM (see Table 1).

In a further experiment, chemotaxis of BASMC (bovine aorta smooth muscle cells) in the presence of the specific compounds of the present invention was tested (protocol described in Palumbo et al., 2004). Modified Boyden chambers were used with filters having 5-8 $\mu$m pore size and treated with collagen I (100 $\mu$g/ml in 0.5 M acetic acid) and fibronectin (10 $\mu$g/ml, Roche). BASMC (bovine aorta smooth muscle cells) were cultured in serum-free DMEM and a sample of 20,000-40,000 cells was added to the upper well of a Boyden chamber.

The compounds were dissolved as described above. HMGB-1 (from calf thymus) concentration was 25 ng/ml, that one of fMLP was 0,1 $\mu$M. Overnight cell migration was allowed at 37+/-0.5 °C, then cells were scraped off and filters were fixed in methanol and stained in a solution of 10% crystal violet in 20% methanol. All experiments were performed at least twice in triplicate. Results are the mean +/- SD of the number of cells counted in 10 high power fields per filters and expressed as folds over control. Random cell migration, i.e. migration in the absence of chemoattractant, was given the arbitrary value of 100%. Statistical analysis was performed using Student's t test for pairwise comparisons of treatment, or an ANOVA model for the evaluation of treatments with increasing concentrations of a reagent. A concentration dependent inhibition of migration of BASMC was observed in the bent molecules of the present invention ($EC_{50} < 3$ nM). The linear control molecules had no effect.

**Example 3**

Stability of bent Nucleic Acids and Nucleic Acid Analogues in Human Serum at 37°C

The stability of CT406, a double-stranded DNA-PNA chimera molecule according to the invention (see Fig. 9) was tested in human serum in comparison with CT403, a double-stranded DNA corresponding to the sequence of CT406 (see Fig. 9).

7$\mu$M CT406 (checked by UV detection) was incubated at 37°C in pure human serum. To follow the degradation of CT406, the diminution of the area of the HPLC peak corresponding to the undegraded molecule was checked after different incubation times in serum. To avoid inaccuracies due to different injection volumes, the peak area was expressed as percentage of the total HPLC profile area.

The non-linear regression analysis curve ($R^2 = 0.99$) of CT406 gave, by interpolation, a 50% degradation time of 9 h. After 12 h at 37°C, the undigested CT406 was still the 41% of the total (see Fig. 13).

In contrast, CT403 has a 40 min half-life, and was completely degraded after 1h 35 min.

**Example 4**

Reversal of LPS-induced endotoxemia in mice

Thirty six male 7 to 8-week-old BALB/c mice were purchased from Charles River (Calco, Italy) and allowed to acclimate for a few days before use. The day of the experiment, all mice were given an $LD_{70-90}$ dose (10 mg/kg i.p. in the right inguinal region) of lipopolysaccharide (LPS from Escherichia coli, strain 0111:B4, dissolved in 0.9% sterile saline). Twelve and 20 h after LPS injection, 18 mice received CT406 (2.73 mg/kg i.p., 10 ml/kg, in the left inguinal region) dissolved in phosphate buffered saline, while the remaining 18 mice received the same volume of vehicle (controls). The selected dose of CT406 corresponds only to 25 times its in vitro $IC_{50}$ (~10 nM) against the HMGB1-induced proliferation of BAEC (bovine aorta endothelial cells) cells. Mortality was monitored and recorded twice a day for up to 48 h.

Results are shown in Fig. 14. At the end of the observation period (48 h), 7 out of 18 mice administered CT406 were still alive, while only 3 out of the 18 control mice were surviving.

**REFERENCES**

Andersson, U., Erlandsson-Harris, H., Yang, H. and Tracey, K.J. (2002) HMGB1 as a DNA-binding cytokine J. Leucocyte Biol., 72: 1084-1091

Czura, C.J., Tracey, K.J. (2003) Targeting high mobility group box 1 as a late acting mediator of inflammation Crit. Care Med., 31: S46-S50

Agresti, A. and Bianchi, M.E. (2003) HMGB-proteins and gene expression Current Opin. In Genetics and Develop., 13: 170-178

Degryse, B., de Virgilio, M. (2003) The nuclear protein HMGB1, a new kind of chemokine ? FEBS Letters, 553: 11-17

Thomas, J.O. (2001) HMGB1 and 2: architectural DNA-binding proteins Biochemical Society Transactions, 29: 395-401

Ferrari, S., Harley, V. H., Pontiggia, A., Goodfellow, P. N., Lovell-Badge, R. and Bianchi, M. E. (1992) SRY, like HMGB1, recognizes sharp angles in DNA The EMBO J., 11: 4497-4506

Pontiggia, A., Negri, A., Beltrame, M. and Bianchi, M.E. (1993) Protein HU binds specifically to kinked DNA Mol.

Biol., 7: 343-350

Scaffidi, P., Misteli, T. and Bianchi, M.E. (2002) Release of chromatin protein HMGB1 by necrotic cells triggers inflammation Nature, 418: 191-195

Bonaldi, T., Talamo, F., Scaffidi, P., Ferrera, D., Porto, A., Bachi, A., Rubartelli, A., Agresti, A. and Bianchi M.E. (2003) Monocytic cells hyperacetylate chromatin protein HMGB1 to redirect it towards Secretion The EMBO Journal, 22: 5551-5560

Taniguchi, N., Kawahara, K., Yone, K., Hashiguchi, T.,Yamakuchi, M., Inoue, K., Yamada, S., Ijiri, K., Matsunaga, S., Nakajima, T., Komiya S. and Maruyama, I. (2003) High mobility group box chromosomal protein 1 plays a role in the pathogenesis of arthritis as a novel cytokine Arthritis and Rheumatism, 48:971-981

Palumbo, R., Sanpaolesi, M., De Marchis, F., Tonlorenzi, R., Colombetti, S., Mondino, A., Cossu, G. and Bianchi, M.E. (2004) Extracellular HMGB1, a signal of tissue damage, induces nesoangioblast migration and proliferation The J. of Cell Biology, 164: 441-4.49

Friedman, S.G., Czura, C., J. and Tracey, K.J. (2003) The gesture life of high mobility group box 1 Current Opinion in Clinical Nutrition and Metabolica Care, 6: 283-287

Yang, H., Wang, H., and Tracey, K. J. (2001) HMGB1 rediscovered as a cytokine Shock, 15: 247-253

Gardella, S., Andrei, C., Ferrera, D., Lotti, L.V., Torrisi, M.R., Bianchi, M.E. And Rubartelli, A. (2002) The nuclear protein HMGB1 is secreted by monocytes via a non-classical, vesicle- mediated secretory pathway. EMBO. Rep., 3: 995-1001

Schmidt, A. M., Yan, S.D., Yan, S. F. and Stern, D. M. (2001) The multiligand receptor RAGE as a progression factor amplifying immune and inflammatory responses J. Clin. Invest., 108: 949-955

Lotze, M. T. and De Marco, R. A. (2003) Editorial overview - Dealing with death: HMGB1 as a novel target for cancer therapy Current Opinion in Investigational Drugs, 4: 1405-1409

Pullerits, R., Jonsson, I. M., Verdreng, M., Bokarewa, M., Andersson, U., Erlandsson-Harris, H. and Tarkowski, A. (2003) High mobility group box chromosomal protein 1, a DNA binding cytokine, induces arthritis Arthritis and Rheumatism, 48: 1693-1700

Kokkola, R., Li, J., Sundenberg, E., Aveberger, A. C., Palmblad, K., Yang, H., Tracey, K. J., Andersson, U. and Erlandsson-Harris, H. (2003) Successful treatment of collagen-induced arthritis in mice and rats by targeting extracellular high mobility group box chromosomal protein 1 activity Arthritis and Rheumatism, 48: 2052-2058

Yan, S. D., Chen, X., Fu, J., Chen, M., Zhu, H., Roher, A., Slattery, T., Zhao, L., Nagashima, M., Morser, J., Migheli, A., Nawroth, P., Stern, D., Schmidt, A. M. (1996) RAGE and amyloid-beta peptide neurotoxicity in Alzheimer's disease Nature, 382: 685-691

## Claims

1. Use of synthetic double-stranded nucleic acid or nucleic acid analogue molecules with a bent shape structure, capable of binding to the HMGB1 protein or to HMGB1 homologous proteins for the manufacture of a medicament for the prevention or treatment of HMGB1-associated pathologies or pathologies associated with HMGB1 homologous proteins, wherein the HMGB1-associated pathologies and the pathologies associated with HMGB1 homologous proteins are pathological conditions mediated by activation of the inflammatory cytokine cascade, which are selected from the group consisting of inflammatory disease, autoimmune disease, systemic inflammatory response syndrome, reperfusion injury after organ transplantation, cardiovascular affections, obstetric and gynecologic disease, infectious disease, allergic and atopic disease, solid and liquid tumor pathologies, transplant rejection diseases, congenital diseases, dermatological diseases, neurological diseases, cachexia, renal diseases, iatrogenic intoxication conditions, metabolic and iodiopathic diseases, and ophthalmological diseases.

**2.** The use of claim 1, wherein the synthetic double-stranded nucleic acid or nucleic acid analogue molecules with a bent shape structure are capable of binding to the non-acetylated or/and acetylated form of HMGB1.

**3.** The use of claim 1, wherein the synthetic double-stranded nucleic acid or nucleic acid analogue molecules with a bent shape structure are capable of binding to HMGB2, HMGB3, HMG-1L10, HMG-4L and SP100-HMG.

**4.** The use of claims 1 to 3, wherein the double-stranded nucleic acid or nucleic acid analogue molecules have base-paired and unpaired portions.

**5.** The use of claim 4, wherein the double-stranded nucleic acid or nucleic acid analogue molecules have a bent DNA or a cruciform DNA structure.

**6.** The use of claim 4, wherein the bent shape structure comprises at least one structural bend, preferably one structural bend.

**7.** The use of any one of claims 1 to 6, wherein the medicament comprises a nucleic acid analogue with at least one non-naturally occurring nucleotide building block.

**8.** The use of claim 7, wherein the non-naturally occurring building block is selected from backbone-, sugar- and nucleobase-modified building blocks and combinations thereof.

**9.** The use of claim 8, wherein the non-naturally occurring building block is selected from PNA building blocks and building blocks as shown in Figure 3.

**10.** The use of any one of claims 1 to 9, wherein the acid analogue molecule is a double-stranded DNA/PNA hybrid molecule, a double-stranded DNA/LNA hybrid molecule, a double-stranded LNA/PNA hybrid molecule, a double-stranded DNA/PNA chimera molecule, a double-stranded DNA/LNA chimera molecule, or/and a double-stranded LNA/PNA chimera molecule.

**11.** The use of any one of claims 1 to 10, wherein the nucleic acid or nucleic acid analogue molecule is a molecule represented by general formula (I):

$$
\begin{array}{ccc}
& Z_1 - Z_r & \\
3'\text{-}(W)_s\text{-}Y_1\ldots Y_n & & Y'_1\ldots Y'_m\text{-}(V)_s\text{-}5' \\
= \quad = \quad = & = \quad = \quad = & \qquad (I) \\
= \quad = \quad = & = \quad = \quad = & \\
5'\text{-}(W')_s\text{-}X_1\ldots X_{n'} & \text{------} & X'_1\ldots X'_m\text{-}(V')_s\text{-}3'
\end{array}
$$

wherein
W, W', X, X', Y, Y', V, V' and Z are independently selected from nucleotide building blocks or nucleotide analogue building blocks;
X and Y, X' and Y', W and W' and V and V', respectively, are each complementary building blocks, preferably matched according to the Watson and Crick base pairing;
$Z_1$ to $Z_r$ represent an extruding loop of unpaired nucleotide building blocks or unpaired nucleotide analogue building blocks;
n, m, n', m' are integers from 2 to 20;
r is an integer from 1 to 10; and
s and s' are each independently an integer from 0 to 10.

**12.** The use of claim 11, wherein W, W', X, X', Y, Y', V, V' and Z are independently selected from DNA nucleotide building blocks, LNA nucleotide building blocks or PNA nucleotide analogue building blocks or sugar-, backbone- and/or nucleobase-modified nucleotide building blocks.

13. The use of claims 11 or 12, wherein Z is an adenine nucleotide or an adenine nucleotide analogue and r is 2 or r = 6.

14. The use of claims 11 or 12, wherein W, X, X', Y, Y' and V are DNA nucleotide building blocks or/and LNA nucleotide building blocks,
W and V' are PNA nucleotide building blocks,
n and m' are 2;
n' and m are 5;
r is 2 or 6;
s is 0 and s' is 3.

15. The use of any one of claims 1 to 14 in combination with at least one further agent capable of inhibiting an early mediator of the inflammatory cytokine cascade.

16. The use of claim 15, wherein the further agent is an antagonist or inhibitor of a cytokine selected from the group consisting of TNF, IL-1$\alpha$, IL-1$\beta$, IL-R$_a$, IL-8, MIP-1$\alpha$, MIF-1$\beta$, MIP-2, MIF and IL-6.

17. The use of claim 15, wherein the further agent is an antibody to RAGE, a nucleic acid or nucleic acid analogue capable of inhibiting RAGE expression, e.g. an antisense molecule, a ribozyme or a RNA interference molecule, or a small synthetic molecule antagonist of the HMGB1 interaction with RAGE.

18. The use of claim 15, wherein the further agent is soluble RAGE (sRAGE).

19. The use of any of claims 1 to 14 in combination with a further agent which is an inhibitor of the interaction of a Toll-like receptor (TLR), in particular of TLR2, TLR4, TLR7, TLR8 or/and TLR9, with HMGB1, preferably a monoclonal or polyclonal antibody, a nucleic acid or nucleic acid analogue capable of inhibiting TLR expression, e.g. an antisense molecule, a ribozyme or a RNA interference molecule, or a synthetic molecule having a size of less than 1000 Dalton.

20. The use of claim 19, wherein the further agent is a known inhibitor of a Toll-like receptor (TLR), in particular of TLR2, TLR4, TLR7, TLR8 or/and TLR9, in particular a nucleic acid or nucleic acid analogue capable of inhibiting TLR expression, e.g. an antisense molecule, a ribozyme or a RNA interference molecule.

21. The use of any of claims 1 to 14 wherein the further agent is the N-terminal lectin-like domain (D1) of thrombomodulin.

22. A pharmaceutical composition comprising an effective amount of at least one double-stranded nucleic acid or nucleic acid analogue molecules of any one of claims 1 to 12 as an active agent and optionally a pharmaceutically acceptable carrier.

23. The composition of claim 22 comprising a pharmaceutically acceptable salt of the at least one double-stranded nucleic acid or nucleic acid analogue molecule selected from salts of inorganic acids, salts of organic acids and cationic salts, and optionally comprising an auxiliary substance.

24. The composition of claims 22 or 23, wherein the double-stranded nucleic acid or nucleic acid analogue molecules are in combination with at least one further agent capable of inhibiting an early mediator of the inflammatory cytokine cascade as defined in claims 15 to 18, or/and with a further agent as defined in claims 19 or 21.

25. The composition of claims 22 or 23 for diagnostic applications.

26. The composition of claim 25 which is a kit for determining HMGB1 in body fluids, preferably serum or/and plasma, obtained from a patient suspected to be affected by an HMGB1-associated pathology, preferably by an inflammatory, cardiovascular, neurodegenerative, neoplastic or/and autoimmune pathology.

27. The composition of any of the claims 22 to 24 for therapeutic applications.

28. The use of synthetic double-stranded nucleic acid or nucleic acid analogue molecules according to any one of claims 1 to 12, wherein said molecules are reversibly immobilised on the surface of medical devices.

29. The use of claim 28, wherein said medical devices are surgical instruments, implants, catheters or stents.

30. Medical device reversibly coated with synthetic double-stranded nucleic acid or nucleic acid analogue molecules according to any one of claims 1 to 12.

31. Medical device of claim 30, wherein the medical device is selected from surgical instruments, implants, catheters or stents.

32. A kit for determining HMGB1 in body fluids, preferably serum or/and plasma, obtained from a patient suspected to be affected by an HMGB1-associated pathology, preferably by an inflammatory, cardiovascular, neurodegenerative, neoplastic or/and autoimmune pathology, comprising at least one double-stranded nucleic acid or nucleic acid analogue molecules of any one of claims 1 to 12.

33. Kit of claim 32, wherein the non-acetylated or/and the acetylated form of HMGB1 or/and of HMGB1 homologous proteins is determined.

## Patentansprüche

1. Verwendung von synthetischen, doppelsträngigen Nukleinsäure- oder Nukleinsäure-analogen Molekülen mit einer bogenförmigen Struktur, die in der Lage sind, an das HMGB1-Protein oder an HMGB1-homologe Proteine zu binden, zur Herstellung eines Medikaments für die Vorbeugung oder Behandlung von mit HMGB1 im Zusammenhang stehenden Pathologien oder Pathologien im Zusammenhang mit HMGB1-homologen Proteinen, worin die mit HMGB1 im Zusammenhang stehenden Pathologien und die Pathologien im Zusammenhang mit HMGB1-homologen Proteinen durch die Aktivierung der inflammatorischen Cytokin-Kaskade vermittelte pathologische Zustände sind, die ausgewählt sind aus der Gruppe bestehend aus Inflammatorischer Erkrankung, Autoimmunerkrankung, Systemischem inflammatorischem Response-Syndrom, Reperfusionsschaden nach Organtransplantation, Herz-Kreislauf-Erkrankungen, Entbindungs- und Gynäkologie-Erkrankung, infektiöse Erkrankung, allergische und atopische Erkrankung, solide und flüssige Tumorpathologien, Transplantatabstoßungserkrankungen, kongenitale Erkrankungen, dermatologische Erkrankungen, neurologische Erkrankungen, Kachexie, Nierenerkrankungen, iatrogene Intoxikationszustände, metabolische und idiopathische Erkrankungen und ophthalmologische Erkrankungen.

2. Verwendung nach Anspruch 1, worin die synthetischen, doppelsträngigen Nukleinsäure- oder Nukleinsäure-analogen Moleküle mit einer bogenförmigen Struktur in der Lage sind, an die nicht-acetylierte oder/und acetylierte Form von HMGB1 zu binden.

3. Verwendung nach Anspruch 1, worin die synthetischen, doppelsträngigen Nukleinsäure- oder Nukleinsäure-analogen Moleküle mit einer bogenförmigen Struktur in der Lage sind, an HMGB2, HMGB3, HMG-1L10, HMG-4L und SP100-HMG zu binden.

4. Verwendung nach Anspruch 1 bis 3, worin die doppelsträngigen Nukleinsäure- oder Nukleinsäure-analogen Moleküle Basen-gepaarte und ungepaarte Abschnitte aufweisen.

5. Verwendung nach Anspruch 4, worin die doppelsträngigen Nukleinsäure- oder Nukleinsäure-analogen Moleküle eine gebogene DNA- oder eine kreuzförmige DNA-Struktur aufweisen.

6. Verwendung nach Anspruch 4, worin die bogenförmige Struktur wenigstens eine strukturelle Biegung, vorzugsweise eine strukturelle Biegung aufweist.

7. Verwendung nach einem der Ansprüche 1 bis 6, worin das Medikament ein Nukleinsäureanalogon mit wenigstens einem nicht-natürlich vorkommenden Nukleotid-Baustein umfasst.

8. Verwendung nach Anspruch 7, worin der nicht-natürlich vorkommende Baustein ausgewählt ist Rückgrat-, Zucker- und Nukleobasen-modifizierten Bausteinen und Kombinationen davon.

9. Verwendung nach Anspruch 8, worin der nicht-natürlich vorkommende Baustein ausgewählt ist aus PNA-Bausteinen und in Figur 3 gezeigten Bausteinen.

10. Verwendung nach einem der Ansprüche 1 bis 9, worin das Nukleinsäure-analoge Molekül ein doppelsträngiges DNA/PNA-Hybridmolekül, doppelsträngiges DNA/LNA-Hybridmolekül, ein doppelsträngiges LNA/PNA-Hybridmolekül, ein doppelsträngiges chimäres DNA/PNA Molekül, ein doppelsträngiges chimäres DNA/LNA Molekül oder/und ein doppelsträngiges chimäres LNA/PNA Molekül ist.

11. Verwendung nach einem der Ansprüche 1 bis 10, worin das Nukleinsäure- oder Nukleinsäure-analoge Molekül ein Molekül ist, das durch die allgemeine Formel (I) wiedergegeben wird:

$$3'\text{-}(W)_s\text{-}Y_1....Y_n \quad \overset{\lceil Z_1 - Z_r \rceil}{\phantom{x}} \quad Y'_1....Y'_m\text{-}(V)_s\text{-}5'$$

$$5'\text{-}(W')_s\text{-}X_1....X_{n'} \quad ----- \quad X'_1....X'_m\text{-}(V')_s\text{-}3' \tag{I}$$

worin

W, W', X, X', Y, Y', V, V' und Z unabhängig ausgewählt sind aus Nukleotid-Bausteinen oder Nukleotid-analogen Bausteinen;

X und Y, X' und Y', W und W' bzw. V und V' jeweils komplementäre Bausteine sind, die vorzugsweise gemäß der Watson und Crick Basenpaarung passen;

$Z_1$ bis $Z_r$ eine vorstehende Schleife ungepaarter Nukleotid-Bausteine oder ungepaarter Nukleotid-analoger Bausteine bedeuten;

n, m, n' und m' ganze Zahlen von 2 bis 20 sind;

r eine ganze Zahl von 1 bis 10 ist; und

s und s' jeweils unabhängig eine ganze Zahl von 0 bis 10 sind.

12. Verwendung nach Anspruch 11, worin W, W, X, X', Y, Y', V, V' und Z unabhängig ausgewählt sind aus DNA-Nukleotidbausteinen, LNA-Nukleotidbausteinen oder PNA-Nukleotid-analogen Bausteinen oder Zucker-, Rückgrat- und/oder Nukleobasen-modifizierten Nukleotidbausteinen.

13. Verwendung nach Anspruch 11 oder 12, worin Z ein Adenin-Nukleotid oder ein Adenin-Nukleotid-Analogon ist, und r 2 ist oder r = 6.

14. Verwendung nach Anspruch 11 oder 12, worin W, X, X', Y, Y' und V DNA-Nukleotidbausteine oder/und LNA-Nukleotidbausteine sind, W und V' PNA-Nukleotidbausteine sind;

n und m' 2 sind;

n' und m 5 sind;

r 2 oder 6 ist;

s 0 ist und s' 3 ist.

15. Verwendung nach einem der Ansprüche 1 bis 14 in Verbindung mit wenigstens einem weiteren Mittel, das in der Lage ist, einen frühen Vermittler der inflammatorischen Cytokin-Kaskade zu hemmen.

16. Verwendung nach Anspruch 15, worin das weitere Mittel ein Antagonist oder Hemmer eines Cytokins ist, das ausgewählt ist aus der Gruppe bestehend aus TNF, IL-1$\alpha$, IL-1$\beta$, IL-R$_a$, IL-8, MIP-1$\alpha$, MIF-1$\beta$, MIP-2, MIF und IL-6.

17. Verwendung nach Anspruch 15, worin das weitere Mittel ein Antikörper gegen RAGE, eine zur Hemmung der RAGE-Expression fähige Nukleinsäure oder ein Nukleinsäureanalogon, z.B. ein Antisense-Molekül, ein Ribozym oder ein RNA-Interferenz-Molekül, oder ein kleiner synthetischer molekularer Antagonist der HMGB1-Wechselwirkung mit RAGE ist.

18. Verwendung nach Anspruch 15, worin das weitere Mittel lösliches RAGE (sRAGE) ist.

19. Verwendung nach einem der Ansprüche 1 bis 14, in Verbindung mit einem weiteren Mittel, das ein Hemmer der Wechselwirkung eines Toll-like Rezeptors (TLR), insbesondere TLR2, TLR4, TLR7, TLR8 oder/und TLR9, mit HMGB1 ist, vorzugsweise ein monoklonaler oder polyklonaler Antikörper, eine zur Hemmung der TLR-Expression geeignete Nukleinsäure oder ein Nukleinsäureanalogon, z.B. ein Antisense-Molekül, ein Ribozym oder ein RNA-Interferenz-Molekül, oder ein synthetisches Molekül mit einer Größe von weniger als 1000 Dalton.

20. Verwendung nach Anspruch 19, worin das weitere Mittel ein bekannter Hemmer eines Toll-like Rezeptors (TLR), insbesondere TLR2, TLR4, TLR7, TLR8 oder/und TLR9, ist, insbesondere eine zur Hemmung der TLR-Expression geeignete Nukleinsäure oder ein Nukleinsäureanalogon, z.B. ein Antisense-Molekül, ein Ribozym oder ein RNA-Interferenz-Molekül.

21. Verwendung nach einem der Ansprüche 1 bis 14, worin das weitere Mittel die N-terminale Lektin-ähnliche Domäne (D1) von Thrombomodulin ist.

22. Pharmazeutische Zusammensetzung, umfassend eine wirksame Menge wenigstens einer doppelsträngigen Nukleinsäure oder eines Nukleinsäure-analogen Moleküls nach einem der Ansprüche 1 bis 12 als Wirkstoff und gegebenenfalls einen pharmazeutisch annehmbaren Träger.

23. Zusammensetzung nach Anspruch 22, umfassend ein pharmazeutisch annehmbares Salz der wenigstens einen doppelsträngigen Nukleinsäure oder des Nukleinsäure-analogen Moleküls, ausgewählt aus Salzen anorganischer Säuren, Salzen organischer Säuren und kationischen Salzen und gegebenenfalls umfassend eine Hilfssubstanz.

24. Zusammensetzung nach Anspruch 22 oder 23, worin die doppelsträngigen Nukleinsäure- oder Nukleinsäure-analogen Moleküle in Kombination mit wenigstens einem weiteren Mittel vorliegen, das zur Hemmung eines frühen Vermittlers der inflammatorischen Cytokin-Kaskade geeignet ist, wie in Anspruch 15 bis 18 definiert, oder/und mit einem weiteren Mittel wie in Anspruch 19 oder 21 definiert.

25. Zusammensetzung nach Anspruch 22 oder 23 für diagnostische Anwendungen.

26. Zusammensetzung nach Anspruch 25, welche ein Kit zur Bestimmung von HMGB1 in Körperflüssigkeiten ist, vorzugsweise Serum oder/und Plasma, das von einem Patienten erhalten wurde, der unter Verdacht steht, von einer Pathologie im Zusammenhang mit HMGB1 betroffen zu sein, vorzugsweise einer entzündlichen, kardiovaskulären, neurodegenerativen, neoplastischen oder/und autoimmunen Pathologie.

27. Zusammensetzung nach einem der Ansprüche 22 bis 24, für therapeutische Anwendungen.

28. Verwendung von doppelsträngigen Nukleinsäure- oder Nukleinsäure-analogen Molekülen nach einem der Ansprüche 1 bis 12, worin die Moleküle reversibel auf der Oberfläche medizinischer Vorrichtungen immobilisiert sind.

29. Verwendung nach Anspruch 28, worin die medizinischen Vorrichtungen chirurgische Instrumente, Implantate, Katheter oder Stents sind.

30. Medizinische Vorrichtung, die reversibel mit einer synthetischen doppelsträngigen Nukleinsäure oder einem Nukleinsäure-analogen Molekül nach einem der Ansprüche 1 bis 12 beschichtet ist.

31. Medizinische Vorrichtung nach Anspruch 30, worin die medizinische Vorrichtung ausgewählt ist aus chirurgischen Instrumenten, Implantaten, Kathetern oder Stents.

32. Kit zur Bestimmung von HMGB1 in Körperflüssigkeiten, vorzugsweise Serum oder/und Plasma, das von einem Patienten erhalten wurde, der unter Verdacht steht, von einer Pathologie im Zusammenhang mit HMGB1 betroffen zu sein, vorzugsweise einer entzündlichen, kardiovaskulären, neurodegenerativen, neoplastischen oder/und autoimmunen Pathologie, umfassend wenigstens eine doppelsträngige Nukleinsäure oder ein Nukleinsäure-analoges Molekül nach einem der Ansprüche 1 bis 12.

33. Kit nach Anspruch 32, worin die nicht-acetylierte oder/und die acetylierte Form von HMGB1 oder/und HMGB1-homologen Proteinen bestimmt wird.

**Revendications**

1. Utilisation de molécules synthétiques d'acides nucléiques ou d'analogues d'acides nucléiques double-brin à structure courbe, capables de se lier à la protéine HMGB1 ou à des protéines homologues de la HMGB1 pour la fabrication d'un médicament destiné à la prévention ou au traitement de pathologies associées à la protéine HMGB1 ou de pathologies associées aux protéines homologues de la HMGB1, les pathologies associées à la protéine HMGB1 et les pathologies associées aux protéines homologues de la HMGB1 correspondant à des états pathologiques médiés par l'activation de la cascade des cytokines inflammatoires, choisis dans le groupe constitué des maladies inflammatoires, des maladies auto-immunes, du syndrome de réponse inflammatoire systémique, des lésions de reperfusion faisant suite à une transplantation d'organe, des affections cardiovasculaires, des maladies obstétriques et gynécologiques, des maladies infectieuses, des affections allergiques et atopiques, des pathologies de type tumeur solide et liquide, des rejets après transplantation, des maladies congénitales, des maladies dermatologiques, des maladies neurologiques, de la cachexie, des maladies rénales, des affections de type intoxication iatrogène, des maladies métaboliques et idiopathiques et des maladies ophtalmologiques.

2. Utilisation selon la revendication 1, dans laquelle les molécules synthétiques d'acides nucléiques ou d'analogues d'acides nucléiques double-brin à structure courbe sont capables de se lier à la forme non acétylée et/ou acétylée de la protéine HMGB1.

3. Utilisation selon la revendication 1, dans laquelle les molécules synthétiques d'acides nucléiques ou d'analogues d'acides nucléiques double-brin à structure courbe sont capables de se lier aux protéines HMGB2, HMGB3, HMG-1L10, HMG-4L et SP 100-HMG.

4. Utilisation selon les revendications 1 à 3, dans laquelle les molécules d'acides nucléiques ou d'analogues d'acides nucléiques double-brin possèdent des régions où les bases sont appariées et d'autres où elles ne le sont pas.

5. Utilisation selon la revendication 4, dans laquelle les molécules d'acides nucléiques ou d'analogues d'acides nucléiques double-brin possèdent une structure de type ADN courbe ou ADN cruciforme.

6. Utilisation selon la revendication 4, dans laquelle la structure de forme courbe comprend au moins une courbure structurale et, de préférence, une courbure structurale.

7. Utilisation selon l'une quelconque des revendications 1 à 6, dans laquelle le médicament comprend un analogue d'acide nucléique comportant au moins une brique nucléotidique non naturelle.

8. Utilisation selon la revendication 7, dans laquelle la brique non naturelle est choisie parmi des briques modifiées au niveau de leur squelette, de leur sucre ou de leur nucléobase, ainsi que leurs combinaisons.

9. Utilisation selon la revendication 8, dans laquelle la brique non naturelle est choisie parmi les briques d'ANP (en anglais PNA) et les briques présentées sur la figure 3.

10. Utilisation selon l'une quelconque des revendications 1 à 9, dans laquelle la molécule analogue d'acide est une molécule hybride d'ADN/ANP double-brin, une molécule hybride d'ADN/LNA double-brin, une molécule hybride de LNA/ANP double-brin, une molécule chimère d'ADN/ANP double-brin, une molécule chimère d'ADN/LNA double-brin et/ou une molécule chimère de LNA/ANP double-brin.

11. Utilisation selon l'une quelconque des revendications 1 à 10, dans laquelle la molécule d'acide nucléique ou d'analogue d'acide nucléique est une molécule de formule générale (I) :

$$Z_1\text{-}Z_r$$

$$3'\text{-}(W)_s\text{-}Y_1\ldots Y_n \qquad Y'_1\ldots Y'_m\text{-}(V)_s\text{-}5'$$

$$= \quad = \quad = \quad \equiv \quad = \quad =$$

$$= \quad = \quad = \quad \equiv \quad = \quad = \qquad \text{(I)}$$

$$5'\text{-}(W')_s\text{-}X_1\ldots X_{n'} \text{-----} X'_1\ldots X'_m\text{-}(V')_s\text{-}3'$$

où

W, W', X, X', Y, Y', V, V' et Z sont choisis indépendamment les uns des autres parmi des briques nucléotidiques ou des briques d'analogues de nucléotides ;

X et Y, X' et Y', W et W' et V et V', représentent, respectivement, des briques complémentaires, de préférence assorties selon l'appariement des bases de Watson et Crick ;

$Z_1$ à $Z_r$ représentent une boucle protubérante de briques nucléotidiques non appariées ou de briques d'analogues de nucléotides non appariées ;

n, m, n' et m' représentent des nombres entiers de 2 à 20 ;

r représente un nombre entier de 1 à 10 ; et

s et s' représentent indépendamment l'un de l'autre un nombre entier de 0 à 10.

**12.** Utilisation selon la revendication 11, dans laquelle W, W', X, X', Y, Y', V, V' et Z sont choisis indépendamment les uns des autres parmi des briques nucléotidiques d'ADN, des briques nucléotidiques de LNA ou des briques d'analogues de nucléotides d'ANP ou des briques nucléotidiques modifiées au niveau de leur sucre, de leur squelette et/ou de leur nucléobase.

**13.** Utilisation selon les revendications 11 ou 12, dans laquelle Z est un nucléotide d'adénine ou un analogue nucléotidique d'adénine et r est égal à 2 ou à 6.

**14.** Utilisation selon les revendications 11 ou 12, dans laquelle W', X, X', Y, Y' et V sont des briques nucléotidiques d'ADN et/ou des briques nucléotidiques de LNA,
W et V' sont des briques nucléotidiques de d'ANP,
n et m' sont égaux à 2 ;
n' et m sont égaux à 5 ;
r est égal à 2 ou à 6 ;
s est égal à 0 et s' est égal à 3.

**15.** Utilisation selon l'une quelconque des revendications 1 à 14 en association avec au moins un agent supplémentaire capable d'inhiber un médiateur précoce de la cascade des cytokines inflammatoires.

**16.** Utilisation selon la revendication 15, dans laquelle l'agent supplémentaire est un antagoniste ou un inhibiteur d'une cytokine choisie dans le groupe constitué par TNF, IL-1$\alpha$, IL-1$\beta$, IL-R$_a$, IL-8, MIP-1$\alpha$, MIF-1$\beta$, MIP-2, MIF et IL-6.

**17.** Utilisation selon la revendication 15, dans laquelle l'agent supplémentaire est un anticorps dirigé contre RAGE, un acide nucléique ou un analogue d'acide nucléique capable d'inhiber l'expression de RAGE, par exemple une molécule antisens, un ribozyme ou une molécule d'ARN d'interférence, ou une petite molécule de synthèse antagoniste de l'interaction de HMGB1 avec RAGE.

**18.** Utilisation selon la revendication 15, dans laquelle l'agent supplémentaire est du RAGE soluble (RAGEs).

**19.** Utilisation selon l'une quelconque des revendications 1 à 14 en association avec un agent supplémentaire qui est un inhibiteur de l'interaction d'un récepteur de type Toll (TLR), en particulier de TLR2, TLR4, TLR7, TLR8 et/ou TLR9, avec HMGB1, de préférence un anticorps monoclonal ou polyclonal, un acide nucléique ou un analogue d'acide nucléique capable d'inhiber l'expression de TLR, par exemple une molécule antisens, un ribozyme ou une molécule d'ARN d'interférence, ou encore une molécule de synthèse d'une taille inférieure à 1 000 daltons.

**20.** Utilisation selon la revendication 19, dans laquelle l'agent supplémentaire est un inhibiteur connu d'un récepteur de type Toll (TLR), en particulier de TLR2, TLR4, TLR7, TLR8 et/ou TLR9, en particulier un acide nucléique ou un analogue d'acide nucléique capable d'inhiber l'expression de TLR, par exemple une molécule antisens, un ribozyme ou une molécule d'ARN d'interférence.

21. Utilisation selon l'une quelconque des revendications 1 à 14, dans laquelle l'agent supplémentaire est le domaine N-terminal de type lectine (D1) de la thrombomoduline.

22. Composition pharmaceutique comprenant une quantité efficace d'au moins une molécule d'acide nucléique ou d'analogue d'acide nucléique double-brin selon l'une quelconque des revendications 1 à 12 en tant que principe actif et, éventuellement, un support acceptable sur le plan pharmaceutique.

23. Composition selon la revendication 22 comprenant un sel acceptable sur le plan pharmaceutique d'au moins une molécule d'acide nucléique ou d'analogue d'acide nucléique double-brin, choisi parmi les sels d'acides inorganiques, les sels d'acides organiques et les sels cationiques, et comprenant, éventuellement, une substance auxiliaire.

24. Composition selon les revendications 22 ou 23, dans laquelle les molécules d'acides nucléiques ou d'analogues d'acides nucléiques double-brin sont associées à au moins un agent supplémentaire capable d'inhiber un médiateur précoce de la cascade des cytokines inflammatoires comme défini dans les revendications 15 à 18 et/ou à au moins un agent supplémentaire comme défini dans les revendications 19 ou 21.

25. Composition selon les revendications 22 ou 23 destinée à des applications de diagnostic.

26. Composition selon la revendication 25 qui est une trousse destinée à déterminer HMGB1 dans des fluides organiques, de préférence du sérum et/ou du plasma, provenant d'un patient chez qui l'on suspecte une pathologie associée à HMGB1, de préférence une pathologie inflammatoire, cardiovasculaire, neurodégénérative, néoplasique et/ou auto-immune.

27. Composition selon l'une quelconque des revendications 22 à 24 destinée à des applications thérapeutiques.

28. Utilisation de molécules synthétiques d'acides nucléiques ou d'analogues d'acides nucléiques double-brin selon l'une quelconque des revendications 1 à 12, dans laquelle lesdites molécules sont immobilisées, de façon réversible, à la surface de dispositifs médicaux.

29. Utilisation selon la revendication 28, lesdits dispositifs médicaux étant des instruments chirurgicaux, des implants, des cathéters ou des stents.

30. Dispositif médical revêtu, de façon réversible, avec des molécules synthétiques d'acides nucléiques ou d'analogues d'acides nucléiques double-brin selon l'une quelconque des revendications 1 à 12.

31. Dispositif médical selon la revendication 30, ledit dispositif médical étant choisi parmi les instruments chirurgicaux, les implants, les cathéters ou les stents.

32. Trousse destinée à déterminer HMGB 1 dans des fluides organiques, de préférence, du sérum et/ou du plasma, provenant d'un patient chez qui l'on suspecte une pathologie associée à HMGB1, de préférence une pathologie inflammatoire, cardiovasculaire, neurodégénérative, néoplasique et/ou auto-immune, comprenant au moins une molécule d'acide nucléique ou d'analogue d'acide nucléique double-brin selon l'une quelconque des revendications 1 à 12.

33. Trousse selon la revendication 32, dans laquelle on peut déterminer la forme non acétylée et/ou acétylée de HMGB 1 et/ou de protéines homologues de HMGB 1.

Table 1

## SUMMARY OF RESULTS: EC50 OF BENT DNA AND DNA/PNA CHIMERAS DETERMINED IN HMGB1 INDUCED CELL MIGRATION AND PROLIFERATION.

| | CT COMPOUND | CHEMOTAXIS EC50 (nM) | PROLIFERATION EC50 (nM) |
|---|---|---|---|
| BENT DNA | CT401 | ~ 3 | ~ 10 |
| | CT402 | < 3 | ~ 30 |
| | CT403 | < 3 | < 10 |
| | CT404 (linear DNA, negative control) | n.d. | n.d. |
| BENT DNA/PNA | CT405 | ~ 5 | < 10 |
| | CT406 | 3 | < 10 |
| | CT407 (linear DNA/PNA chimera, negative control) | n.d. | n.d. |

n.d. = not determinable

Figure 1

(A)

(B)

(C)

(D)

(E)

Figure 2

Figure 3

Figure 4

Figure 5

Figure 6

## Figure 7

Figure 8

Figure 9

**CT401**         CT402         CT403         CT404

CT405         CT406         CT407

## Figure 10

BAEC PROLIFERATION

Legend:
- SERUM FREE
- HMGB1 25 ng/mL
- H+CT401 100 nM
- H+CT402 100 nM
- H+CT403 100 nM
- H+CT404 100 nM

BAEC PROLIFERATION

Legend:
- SERUM FREE
- HMGB1 25 ng/mL
- H+CT401 10 nM
- H+CT402 10 nM
- H+CT403 10 nM
- H+CT404 10 nM

## Chemotaxis assay:

Figure 11

## Chemotaxis assay:

Figure 12

**Figure 13**

CT406 Stability in Human Serum at 37 °C

Human Serum Stability

**HPLC Data:**

| Time (minutes) | Peak Area % | Half-life: 9 h | At 12 h: 41% of the total CT406 is still undigested |
|---|---|---|---|
| 0 | 6.70 | | |
| 30 | 6.48 | | |
| 60 | 6.12 | | |
| 120 | 6.12 | | |
| 180 | 4.89 | | |
| 240 | 4.70 | | |
| 300 | 4.63 | | |
| 1440 | 1.36 | | |
| 2400 | 0.82 | | |

36

Figure 14

LPS challenge

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6468533 B
- WO 02074337 A
- US 20030144201 A

**Non-patent literature cited in the description**

- **ANDERSSON, U. ; ERLANDSSON-HARRIS, H. ; YANG, H. ; TRACEY, K.J.** HMGB1 as a DNA-binding cytokine. *J. Leucocyte Biol.,* 2002, vol. 72, 1084-1091
- **CZURA, C.J. ; TRACEY, K.J.** Targeting high mobility group box 1 as a late acting mediator of inflammation. *Crit. Care Med.,* 2003, vol. 31, S46-S50
- **AGRESTI, A. ; BIANCHI, M.E.** HMGB-proteins and gene expression Current Opin. *Genetics and Develop.,* 2003, vol. 13, 170-178
- **DEGRYSE, B. ; DE VIRGILIO, M.** The nuclear protein HMGB1, a new kind of chemokine ?. *FEBS Letters,* 2003, vol. 553, 11-17
- **THOMAS, J.O.** HMGB1 and 2: architectural DNA-binding proteins. *Biochemical Society Transactions,* 2001, vol. 29, 395-401
- **FERRARI, S. ; HARLEY, V. H. ; PONTIGGIA, A. ; GOODFELLOW, P. N. ; LOVELL-BADGE, R. ; BIANCHI, M. E.** SRY, like HMGB1, recognizes sharp angles in DNA. *The EMBO J.,* 1992, vol. 11, 4497-4506
- **PONTIGGIA, A. ; NEGRI, A. ; BELTRAME, M. ; BIANCHI, M.E.** Protein HU binds specifically to kinked DNA. *Mol. Biol.,* 1993, vol. 7, 343-350
- **SCAFFIDI, P. ; MISTELI, T. ; BIANCHI, M.E.** Release of chromatin protein HMGB1 by necrotic cells triggers inflammation. *Nature,* 2002, vol. 418, 191-195
- **BONALDI, T. ; TALAMO, F. ; SCAFFIDI, P. ; FERRERA, D. ; PORTO, A. ; BACHI, A. ; RUBARTELLI, A. ; AGRESTI, A. ; BIANCHI M.E.** Monocytic cells hyperacetylate chromatin protein HMGB1 to redirect it towards Secretion. *The EMBO Journal,* 2003, vol. 22, 5551-5560
- **TANIGUCHI, N. ; KAWAHARA, K. ; YONE, K. ; HASHIGUCHI, T. ; YAMAKUCHI, M. ; INOUE, K. ; YAMADA, S. ; IJIRI, K. ; MATSUNAGA, S. ; NAKAJIMA, T.** High mobility group box chromosomal protein 1 plays a role in the pathogenesis of arthritis as a novel cytokine. *Arthritis and Rheumatism,* 2003, vol. 48, 971-981
- **PALUMBO, R. ; SANPAOLESI, M. ; DE MARCHIS, F. ; TONLORENZI, R. ; COLOMBETTI, S. ; MONDINO, A. ; COSSU, G. ; BIANCHI, M.E.** Extracellular HMGB1, a signal of tissue damage, induces nesoangioblast migration and proliferation. *The J. of Cell Biology,* 2004, vol. 164, 441-4.49
- **FRIEDMAN, S.G. ; CZURA, C., J. ; TRACEY, K.J.** The gesture life of high mobility group box 1 Current Opinion. *Clinical Nutrition and Metabolica Care,* 2003, vol. 6, 283-287
- **YANG, H. ; WANG, H. ; TRACEY, K. J.** *HMGB1 rediscovered as a cytokine Shock,* 2001, vol. 15, 247-253
- **GARDELLA, S. ; ANDREI, C. ; FERRERA, D. ; LOTTI, L.V. ; TORRISI, M.R. ; BIANCHI, M.E. ; RUBARTELLI, A.** The nuclear protein HMGB1 is secreted by monocytes via a non-classical, vesicle- mediated secretory pathway. *EMBO. Rep.,* 2002, vol. 3, 995-1001
- **SCHMIDT, A. M. ; YAN, S.D. ; YAN, S. F. ; STERN, D. M.** The multiligand receptor RAGE as a progression factor amplifying immune and inflammatory responses. *J. Clin. Invest.,* 2001, vol. 108, 949-955
- **LOTZE, M. T. ; DE MARCO, R. A.** Editorial overview - Dealing with death: HMGB1 as a novel target for cancer therapy. *Current Opinion in Investigational Drugs,* 2003, vol. 4, 1405-1409
- **PULLERITS, R. ; JONSSON, I. M. ; VERDRENG, M. ; BOKAREWA, M. ; ANDERSSON, U. ; ERLANDSSON-HARRIS, H. ; TARKOWSKI, A.** High mobility group box chromosomal protein 1, a DNA binding cytokine, induces arthritis. *Arthritis and Rheumatism,* 2003, vol. 48, 1693-1700
- **KOKKOLA, R. ; LI, J. ; SUNDENBERG, E. ; AVEBERGER, A. C. ; PALMBLAD, K. ; YANG, H. ; TRACEY, K. J. ; ANDERSSON, U. ; ERLANDSSON-HARRIS, H.** Successful treatment of collagen-induced arthritis in mice and rats by targeting extracellular high mobility group box chromosomal protein 1 activity. *Arthritis and Rheumatism,* 2003, vol. 48, 2052-2058

- **YAN, S. D. ; CHEN, X. ; FU, J. ; CHEN, M. ; ZHU, H. ; ROHER, A. ; SLATTERY, T. ; ZHAO, L. ; NAGASHIMA, M. ; MORSER, J.** RAGE and amyloid-beta peptide neurotoxicity in Alzheimer's disease. *Nature,* 1996, vol. 382, 685-691